# EUROPEAN PATENT APPLICATION

(11) **EP 1 464 651 A1**
(43) Date of publication of application: **06.10.2004**
(21) Application number: 03007680.6
(22) Date of filing: 03.04.2003
(51) Int. Cl.: C07K 14/435, C12N 15/12, C12N 15/63, C12N 15/74, C12N 15/79, A01K 67/027, A61K 38/17, A61K 48/00, C12Q 1/68, G01N 33/53

(54) **Dystrophin-related protein (Drop1), a marker for carcinomas**

(71) Applicant: Deutsches Krebsforschungszentrum Stiftung des öffentlichen Rechts, 69120 Heidelberg (DE); Ruprecht-Karls-Universität Heidelberg, 69120 Heidelberg (DE)
(72) Inventor: Ponstingl, Herwig, 69120 Heidelberg (DE); Zimmermann, Hans-Peter, 69214 Heidelberg (DE); Marmé, Alexander, 69117 Heidelberg (DE); Bastert, Gunther, 69115 Heidelberg (DE); Kurek, Raffael, Universitäts-Frauenklinik Tübingen, 72076 Tübingen (DE); Wallwiener, Diethelm, Uni.-Frauenklinik Tübingen, 72076 Tübingen (DE); Moldenhauer, Gerhard, 69120 Heidelberg (DE)
(74) Representative: Schüssler, Andrea, Dr.

(57) **Abstract**

Described is a novel marker protein associated with epithelial tumors, e.g., ovarian, mammary, stomach, kidney, thyroid and lung carcinomas, Drop1, a dystrophin-related protein as well as a nucleic acid molecule encoding Drop1. Furthermore, various diagnostic and therapeutic uses based on the finding that the expression of Drop1 is downregulated in epithelial tumors are described.

SEQUENCE LISTING

<110> Deutsches Krebsforschungszentrum Stiftung des öffentlichen
      Rechts

<120> Drop1, a novel marker for carcinomas

<130> K 3141EP

<140> EP 03007680.6
<141> 2003-04-03

<160> 30

<170> PatentIn version 3.2

<210> 1
<211> 10
<212> DNA
<213> Artificial

<220>
<223> Arbitrary decamer primer

<400> 1
acaaggcacc                                                              10


<210> 2
<211> 10
<212> DNA
<213> Artificial

<220>
<223> Decamer anchor primer

<400> 2
ttttttttcg                                                              10


<210> 3
<211> 24
<212> DNA
<213> Artificial

<220>
<223> Drop1 Upper Primer

<400> 3
ctggagaaac ggctgtcaca aata                                              24


<210> 4
<211> 28
<212> DNA
<213> Artificial

<220>
<223> Drop1 Lower primer

<400> 4
cctctctaca tagtcattcc attggcta                                          28


<210> 5
<211> 33
<212> DNA
<213> Artificial

<220>
<223>  Primer U278

<400>  5
gtcgacgatg gcaacctcca gaggggcctc ccg                                    33


<210>  6
<211>  31
<212>  DNA
<213>  Artificial

<220>
<223>  Primer L1492

<400>  6
gggccctggt gcccagaggt gcaggaagag a                                      31


<210>  7
<211>  33
<212>  DNA
<213>  Artificial

<220>
<223>  Primer

<400>  7
gatatcatgt atttagatgc ggtccacgag ttc                                    33


<210>  8
<211>  34
<212>  DNA
<213>  Artificial

<220>
<223>  Primer

<400>  8
gtcgactggg gacccttgat ttagaggtta actt                                   34


<210>  9
<211>  10001
<212>  DNA
<213>  Homo sapiens

<400>  9
cggccgccgg ccgcagcggg ctgagattgt tgtcctctgt caccagggcg gctgggctcc      60

cgcagtcctg cagaccgcgc ccgatcccgg cgacagggcg ggcggacagc cgcgcatccc     120

cggggtcccg ccgagcctgg gcgcagagag ccgggaggaa gcgttcgctc gcttcgcctt     180

gctgctggga aactgaacga ggccgagaga gaaggttctt gagttcatgt aagaggacag     240

tcttaaaacg gaagaagaaa aagaagcagt tcagtctttg ggagagctgc ctccttgttg     300

agtgctgcaa aggcctggaa ttcatttatg acagaataga tctagaaaag tccaagcatg     360

ttttctagag tggtgtagcc ctgtgctgcc tccagtgaag agtctcttgg tgttggcttc     420

gtgcttccgg agggaccatg gcaacctcca gaggggcctc ccggtgtcct cgggatatcg     480

ccaatgtgat gcagaggctg caagatgagc aagagatagt acaaaaacga actttcacaa     540

aatggatcaa ctctcatctg gccaagcgga aacctccaat ggtggtggac gatctttttg 600
aagacatgaa agatggtgtt aaactgcttg cccttctgga ggtcctgtct gggcagaaac 660
tgccttgtga acaaggacgc cggatgaagc gaatccatgc tgtggctaac attggcacgg 720
cactcaagtt cctcgaagga agaaagtcca tgcacagagg atcaccgatt aaattagtca 780
acattaactc caccgatata gctgatggcc gaccctcaat agttcttgga ttgatgtgga 840
ccattattct atatttccag attgaagagt tgaccagcaa cctgccccag ctccagtctt 900
tgtccagcag cgcatcctcc gtggacagca tagttagctc tgagactccc agcccaccaa 960
gtaaacggaa ggtgaccacc aagatccaag gaaatgctaa gaaggcttta ttaaagtggg 1020
ttcagtacac agctggcaag cagactggaa tagaagtaaa agattttggg aagagttgga 1080
gaagcggggt tgcctttcat tcagttattc atgccattcg accggaattg gtggacttgg 1140
agacagtgaa aggcagatcc aaccgagaaa atttggagga tgctttcact atcgccgaaa 1200
cagaactggg gatcccaaga ctgctagatc ctgaagacgt tgatgtggat aaaccagatg 1260
agaaatctat tatgacctat gtagcccagt ttctgaaaca ttatcctgac atccacaatg 1320
caagcactga tgggcaagag gatgatgaaa tacttccagg tttcccatct tttgcaaatt 1380
ctgtacaaaa ttttaagaga gaagacagag taatttttaa ggaaatgaaa gtttggatag 1440
aacaatttga gagagatttg acaagagcac agatggtgga atcaaattta caggataaat 1500
atcagtcatt taagcacttc agagttcaat atgaaatgaa gaggaaacag attgaacatt 1560
taatacaacc attacacaga gacggtaaat tgtcacttga ccaagcattg gtaaaacaat 1620
cttgggatag agtgacctcc aggctctttg actggcatat acagcttgat aaatctcttc 1680
ctgcacctct gggcaccata ggtgcctggc tgtacagagc ggaggtggcc ctgagagagg 1740
aaataaccgt tcaacaggtc cacgaggaaa cagcaaacac gatacaacgg aaacttgagc 1800
aacataagga tctgcttcaa aacacggatg cccacaaaag agcattccat gaaatctacc 1860
ggaccaggtc tgttaacggg attccagtgc cacctgatca attagaggac atggccgaga 1920
ggtttcattt tgtttcctcc acatcagagc tacacctaat gaaaatggaa tttttagaat 1980
taaagtaccg tctgctctca ctgctggttc ttgcagagtc aaagctgaag tcttggatca 2040
ttaagtacgg gaggagagag tcagtggagc agcttctaca aaactacgtg tcttttatag 2100
aaaatagcaa gttctttgaa caatatgagg tgacatacca gatcttgaaa cagacagctg 2160
agatgtatgt caaagcagat ggttcagtgg aagaagctga gaatgtgatg aaattcatga 2220
atgaaaccac cgctcagtgg aggaatctct cagtagaagt gaggagtgtg aggagcatgc 2280
tggaagaagt gatctctaac tgggatcgct atggcaatac agtggctagt ctgcaagcct 2340
ggctagagga tgctgaaaaa atgctcaatc aatcagaaaa tgccaaaaag gatttttttc 2400
gaaatttacc tcattggatt cagcagcata ctgccatgaa cgatgctggc aattttctaa 2460
ttgaaacctg tgatgagatg gtttcccgtg acctgaagca gcaattactg ttgctaaatg 2520
ggcggtggag ggagttgttt atggaagtca agcaatatgc tcaagctgat gagatggaca 2580

gaatgaagaa ggaatacaca gactgtgttg ttaccctgtc tgcttttgca acggaagccc 2640
ataagaaact ttctgaaccc ttagaagtct cttttatgaa tgtcaagcta ttaattcaag 2700
acttggagga tattgagcag agggtgcctg tgatggatgc ccaatacaag ataattacaa 2760
agacagcaca cctcattacc aaagaaagcc cccaagaaga aggaaaagaa atgtttgcga 2820
ccatgtcaaa gctcaaagag cagctaacca aggtcaaaga atgttactcc ccactccttt 2880
atgagtctca gcagctgttg attccgttgg aggaattaga aaagcagatg acgtcctttt 2940
atgactcact tgggaaaatc aatgaaatta tcacagttct tgagcgtgag gcacaatcga 3000
gtgccctttt taaacaaaaa catcaggaac tgttagcttg tcaagaaaac tgtaagaaaa 3060
ccttgacact tattgagaaa ggcagtcaaa gtgttcaaaa gtttgtgacc ttgagcaacg 3120
tgttaaagca ttttgatcag acgaggctac aaagacagat tgcagatatt catgttgctt 3180
ttcagagtat ggtaaagaaa actggagatt ggaagaagca tgtggaaacc aacagtcgct 3240
tgatgaagaa gtttgaggag tctcgagcag agttggagaa ggtactgcgg attgctcagg 3300
agggcctgga ggaaaagggg gatccagagg agctcctgcg gagacacact gagtttttca 3360
gtcagctgga tcagagggtg ctcaatgctt tcctgaaagc ttgtgatgaa ctcaccgaca 3420
tccttccaga gcaggagcag caggggctgc aggaagctgt tcgaaagctc cacaaacaat 3480
ggaaggatct tcaaggagaa gccccttatc atttgcttca tctgaagatt gatgtggaga 3540
agaataggtt cttagcctct gtagaagaat gcagaactga gctggatcga gagaccaagc 3600
tgatgcccca ggaaggcagt gaaaagataa ttaaagagca cagggttttc ttcagtgaca 3660
aaggtcctca tcatctctgt gagaaaaggt tacagctcat cgaggaactc tgtgtgaaac 3720
tcccagtgcg ggacccagta agggacacac ctggaacctg tcacgtgact ctcaaagagc 3780
tcagagctgc cattgacagc acctacagga agctcatgga agacccagac aagtggaagg 3840
actacactag cagattctct gagttctcat cttggatatc tacaaatgag acacaattaa 3900
aggggatcaa gggtgaggcc atcgatactg ccaaccacgg agaggttaaa cgtgccgttg 3960
aagagatcag aaatggtgtt accaaaaggg gtgagaccct cagctggctg aaatccaggc 4020
tgaaagtttt gacagaagtt tcttctgaga atgaagccca aaagcaggga gatgagctgg 4080
caaaattatc cagctctttc aaggctcttg tgacgctgct gtcagaggtt gaaaagatgc 4140
taagcaattt tggggactgt gtccagtaca aagaaatagt caaaaattct ctcgaagaat 4200
taatttctgg ctctaaagaa gtccaggaac aagctgagaa gatcttggat actgaaaatc 4260
tgtttgaagc acagcagtta cttcttcatc accagcaaaa gacaaagcgg atctcagcaa 4320
agaagagaga tgtgcagcag cagatcgcgc aggcgcagca gggagaaggg gggctgcctg 4380
accgaggcca cgaggagctg cggaagctgg agagcacact ggatggcctg gagcgcagcc 4440
gggagaggca ggaacgccgc atccaggtca cattaagaaa atgggagcga tttgaaacaa 4500
acaaagaaac agtagtaaga tacctttttc aaacaggttc cagtcatgaa cgcttcttga 4560
gttttagcag tttggaaagt ttatcttcag aactggaaca aacaaaggag ttttctaaac 4620

ggacagaaag tattgcagtc caggctgaga accttgtaaa ggaagcttca gagataccgc 4680
ttgggcccca aaataagcag ctgcttcaac agcaggccaa gtcaatcaaa gaacaagtca 4740
aaaaattaga agacacgctt gaagaagata ttaaaaccat ggaaatggtg aaaaccaagt 4800
gggatcattt tggcagtaat tttgagactc tgtccgtctg gataactgag aaagaaaaag 4860
aactcaatgc cttggaaact tcgtcatctg ccatggacat gcaaatcagc caaattaagg 4920
tcacaattca ggaaatagaa agtaagctca gcagcattgt aggattagaa gaagaagccc 4980
agtcttttgc tcagtttgtt accactggag aatctgctcg aattaaagcc aagttgacac 5040
aaataagaag atacggggaa gagcttcgag agcatgcaca gtgtctggaa ggaacaatcc 5100
tgggacattt atctcagcag caaaagtttg aagagaacct tagaaagatc cagcaatctg 5160
tgtctgaatt tgaagataaa cttgctgttc caattaaaat atgttcttca gctacagaaa 5220
catacaaagt tcttcaagaa catatggatc tctgccaggc cctggagtca ctgagcagcg 5280
cgatcactgc cttctcagcc agtgccagga aggttgtgaa cagagattcc tgtgttcagg 5340
aggctgcggc tctacagcag caatacgagg acatcctaag gagggcgaag gagagacaga 5400
cggcgctgga gaatctgctg gcccactggc agaggctaga gaaagaacta tcatcctttt 5460
tgacctggtt agagcggggt gaagctaaag ccagttcccc agaaatggac atttctgcag 5520
acagagtcaa agtggaaggt gaacttcagt taatacaggc actgcaaaat gaagttgtat 5580
cccaggcctc attctatagc aaacttttgc aattgaagga atcattgttc tcagtagcct 5640
ccaaagatga tgtgaaaatg atgaaactac atttggagca gttggatgag agatggagag 5700
atttaccaca gatcattaac aaaaggatta attttcttca gtctgtggtt gctgaacacc 5760
agcaatttga tgagctgctg ctttcctttt ctgtctggat taaactgttt ctcagtgaat 5820
tacaaactac ctctgagatt agcataatgg accatcaagt agcccttact cggcataagg 5880
accacgcagc agaagtagag agcaaaaagg gcgaattgca gagtctgcag ggtcacttag 5940
caaagttggg ttctctgggc cgtgctgagg acctccacct cctgcaggga aaggctgagg 6000
actgcttcca gctgtttgag gaggccagcc aggttgtgga gaggcggcag cttgccctgt 6060
cccatttggc agaattcctc cagagccatg cctctctgtc cggcattctc cgccagctga 6120
ggcaaacagt ggaagcaacc aacagtatga ataagaacga gtctgatttg atagaaaagg 6180
acctcaatga tgctcttcaa aatgctaaag cattagaatc tgctgccgtc agtctggatg 6240
gcattctttc caaagcccaa taccatctga aaatcgggag ctctgagcaa aggacttcct 6300
gcagagccac ggctgatcag ctctgtggag aggtagagag gatccagaac cttctgggaa 6360
ccaagcagag tgaggcagat gctctggcag tgttgaaaaa agcattccaa gaccagaaag 6420
aggagcttct gaaaagcatt gaggacattg aagaaaggac tgacaaagag cgattgaaag 6480
aacctacccg ccaagctctt cagcagaggt taagagtgtt taatcagcta gaagatgaat 6540
tgaattctca cgagcatgaa ctatgttggt tgaaagacaa agccaagcaa attgcccaga 6600
aagatgtagc ttttgcacct gaagttgaca gggagataaa ccgcttagag gtcacctggg 6660

atgataccaa aagactaatt catgaaaatc agggtcagtg ctgtggactt attgacttaa 6720
tgagagaata tcagaacctg aaatcagctg tatctaaagt cttagaaaat gccagcagtg 6780
tgattgtaac cagaactacc ataaaagatc aggaggatct taaatgggct ttttccaagc 6840
atgaaactgc caagaacaaa atgaattaca aacagaaaga cttggataac tttaccagca 6900
aaggaaaaca cttgttatct gagctgaaga aaattcacag tagtgatttc agcttggtga 6960
aaacagacat ggagagcacc gtggacaaat ggctggatgt atcagagaaa cttgaagaaa 7020
acatggatag gctgagagta agcctgtcca tttgggatga tgtactgtca actagagatg 7080
agattgaggg atggtcaaac aactgcgttc cacagatggc agaaaacatc agcaacctgg 7140
ataaccacct cagagctgaa gaactgctta aagaatttga gtctgaagtt aaaaacaaag 7200
cattgagatt ggaagaactg cattccaaag ttaatgatct gaaagaatta actaaaaatc 7260
tagaaacacc gccagacctt cagtttatag aagcagactt aatgcagaaa ctggagcatg 7320
ccaaagaaat aactgaagta gcaaaaggaa ccctgaagga tttcacggct caaagtacac 7380
aagtggagaa gtttattaat gacataacaa catggttcac aaaagtggaa gaatcgttga 7440
tgaactgtgc ccaaaatgag acttgtgaag cattgaaaaa agtcaaggat atacaaaaag 7500
aacttcaaag tcaacaaagc aacatcagct ctacccaaga aaatctcaat agcttgtgcc 7560
gcaagtacca ctcagctgag ttggagagcc tgggccgtgc aatgactggt ctgataaaga 7620
aacatgaagc cgtgagccag ttgtgctcca aaacccaggc cagcctgcag gaatctctgg 7680
aaaaacactt cagtgagtct atgcaggaat tccaagaatg gtttttggga gcaaaggcag 7740
cagcaaaaga atcatcagat cgcaccggtg acagcaaagt tctagaagca aagctccatg 7800
atcttcagaa cattttggac tcagtcagtg atgggcagag caaacttgat gcagtgactc 7860
aagaaggaca aactttgtat gcacatttgt ctaaacaaat tgtcagtagc attcaagaac 7920
aaatcacaaa ggccaatgaa gagtttcaag catttctgaa acaatgcctt aaagataagc 7980
aggctcttca agactgtgct tcagaacttg gaagctttga agatcagcac agaaaactga 8040
acttatggat ccatgaaatg gaagaaaggt tcaatacgga aaacttggga gagagtaaac 8100
agcacattcc tgagaagaaa aatgaagttc ataaagttga aatgtttttg ggagaactgc 8160
tggctgcaag agagtctctt gataagcttt cccagagagg gcagcttctg agtgaagaag 8220
gccacggtgc tgggcaggag ggccgcctgt gttcccagct cctcacaagc caccagaacc 8280
tacttagaat gaccaaagag aaactccgga gctgccaggt ggcccttcag gagcacgaag 8340
ccctggagga agcactgcaa agcatgtggt tctgggtgaa ggccattcag gacagactgg 8400
cctgtgcaga gagcactctt gggagcaaag acaccctgga gaaacggctg tcacaaatac 8460
aggatattct cctgatgaaa ggtgaagggg aagttaagtt gaatatggcc attggcaagg 8520
gggaacaggc cttgagaagt agcaacaaag aaggtcagag ggtgattcag actcagttag 8580
agacccttaa agaagtgtgg gctgacatca tgagctcctc cgtccacgct caaagcactt 8640
tagagtctgt gattagccaa tggaatgact atgtagagag gaaaaaccag ttggagcagt 8700

ggatggaatc agtggatcaa aaaatagaac atcccttaca accacagcca ggtctgaaag 8760
agaagttcgt cctgcttgac cacctccagt ccatcctgtc tgaggcagaa gatcacacga 8820
gagcccttca ccgtctaatt gcgaagtcca gggagctcta cgaaaagaca gaggatgagt 8880
ctttcaagga cacagctcaa gaggagctga aaacacagtt taatgatata atgactgttg 8940
ccaaggaaaa aatgaggaaa gtggaagaga ttgtgaaaga tcatctaatg tatttagatg 9000
cggtccacga gttcacagat tggctccatt cagcaaagga agaacttcac cggtggtcag 9060
atatgtctgg agattcatca gccacccaga aaaagttatc aaaaattaag gagctgatag 9120
attccagaga gattggtgca agccgtctca gcagagtgga gtcgctggct cccgaagtga 9180
aacagaacac aactgccagt gggtgtgagc tcatgcacac ggagatgcag gccctgcgtg 9240
ccgactggaa gcagtgggaa gacagtgtat tccaaacgca gagctgtttg gagaacctgg 9300
tcagccagat ggccctttcg gagcaggaat tctcaggcca agtggctcaa ctggagcagg 9360
ccctggaaca gttcagtgcc cttctgaaaa cctgggctca gcagttaacc ctcctggaag 9420
gcaagaacac ggatgaggag atagtggaat gctggcacaa aggacaagag atactggatg 9480
ctttgcaaaa agcagagcct agaacagagg atctcaagtc tcagctgaat gaactttgtc 9540
gattttccag agacctgagt acctacagtg gaaaagtttc tggcttgatt aaagagtata 9600
attgtctttg tttgcaagca tccaagggct gccagaataa agaacagatt ttacagcaaa 9660
gatttcgaaa ggccttcagg gatttccagc agtggttggt taatgcaaaa atcactaccg 9720
ccaagtgttt tgatatacct caaaatataa gtgaagtttc aactagtctt cagaaaatac 9780
aggagttttt gtcagaaagt gaaaatggac agcacaagct aaacatgatg ctgtctaaag 9840
gggaacttct gagtaccctg ctgaccaaag agaaagcgaa agggatccag gccaaagtta 9900
cagctgcaaa agaagattgg aaaaattttc attcaaatct ccaccaaaaa gaatctgctc 9960
tagagaatct aaagatccaa atgaaggact ttgaagtaag h 10001

<210> 10
<211> 1746
<212> DNA
<213> homo sapiens

<400> 10
cgttcgctcg cttcgccttg ctgctgggaa actgaacgag gccgagagag aaggttcttg 60
agttcatgta agaggacagt cttaaaacgg aagaagaaaa agaagcagtt cagtctttgg 120
gagagctgcc tccttgttga gtgctgcaaa ggcctggaat tcatttatga cagaatagat 180
ctagaaaagt ccaagcatgt tttctagagt ggtgtagccc tgtgctgcct ccagtgaaga 240
gtctcttggt gttggcttcg tgcttccgga gggaccatgg caacctccag aggggcctcc 300
cggtgtcctc gggatatcgc caatgtgatg cagaggctgc aagatgagca agagatagta 360
caaaaacgaa ctttcacaaa atggatcaac tctcatctgg ccaagcggaa acctccaatg 420
gtggtggacg atctttttga agacatgaaa gatggtgtta aactgcttgc ccttctggag 480
gtcctgtctg ggcagaaact gccttgtgaa caaggacgcc ggatgaagcg aatccatgct 540

gtggctaaca ttggcacggc actcaagttc ctcgaaggaa gaaagattaa attagtcaac 600

attaactcca ccgatatagc tgatggccga ccctcaatag ttcttggatt gatgtggacc 660

attattctat atttccagat tgaagagttg accagcaacc tgccccagct ccagtctttg 720

tccagcagcg catcctccgt ggacagcata gttagctctg agactcccag cccaccaagt 780

aaacggaagg tgaccaccaa gatccaagga aatactaaga aggctttatt aaagtgggtt 840

cagtacacag ctggcaagca gactggaata gaagtaaaag attttgggaa gagttggaga 900

agcggggttg cctttcattc agttattcat gccattcgac cggaattggt ggacttggag 960

acagtgaaag gcagatccaa ccgagaaaat ttggaggatg ctttcactat cgccgaaaca 1020

gaactgggga tcccaagact gctagatcct gaagacgttg atgtggataa accagatgag 1080

aaatctatta tgacctatgt agcccagttt ctgaaacatt atcctgacat ccacaatgca 1140

agcactgatg ggcaagagga tgatgaaata cttccaggtt tcccatcttt tgcaaattct 1200

gtacaaaatt ttaagagaga agacggagta atttttaagg aaatgaaagt ttggatagaa 1260

caatttgaga gagatttgac aagagcacag atggtggaat caaatttaca ggataaatat 1320

cagtcattta agcacttcag agttcaatat gaaatgaaga ggaaacagat tgaacattta 1380

atacaaccat tacacagaga cggtaaattg tcacttgacc aagcattggt aaaacaatct 1440

tgggatagag tgacctccag gctctttgac tggcatatac agcttgataa atctcttcct 1500

gcacctctgg gcaccatagg tgcctggctg tacagagcgg aggtggccct gagagaggaa 1560

ataaccgttc aacaggtcca cgaggaaaca gcaaacacga tacaacggaa acttgagcaa 1620

cataaggatc tgcttcaaaa cacggatgcc cacaaaagag cattccatga aatctaccgg 1680

accaggtctg ttaacgggat tccagtgcca cctgatcaat tagaggacat ggccgagagg 1740

tttcat 1746

<210> 11
<211> 53
<212> DNA
<213> Homo sapiens

<400> 11
cgttcgctcg cttcgccttg ctgctgggaa actgaacgag gccgagagag aag 53

<210> 12
<211> 1202
<212> DNA
<213> Homo sapiens

<400> 12
gtcgacgatg gcaacctcca gaggggcctc ccggtgtcct cgggatatcg ccaatgtgat 60

gcagaggctg caagatgagc aagagatagt acaaaaacga actttcacaa aatggatcaa 120

ctctcatctg gccaagcgga aacctccaat ggtggtggac gatctttttg aagacatgaa 180

agatggtgtt aaactgcttg cccttctgga ggtcctgtct gggcagaaac tgccttgtga 240

acaaggacgc cggatgaagc gaatccatgc tgtggctaac attggcacgg cactcaagtt 300

cctcgaagga agaaagatta aattagtcaa cattaactcc accgatatag ctgatggccg 360

accctcaata gttcttggat tgatgtggac cattattcta tatttccaga ttgaagagtt 420

gaccagcaac ctgccccagc tccagtcttt gtccagcagc gcatcctccg tggacagcat 480

agttagctct gagactccca gcccaccaag taaacggaag gtgaccacca agatccaagg 540

aaatgctaag aaggctttat taaagtgggt tcagtacaca gctggcaagc agactggaat 600

agaagtaaaa gattttggga agagttggag aagcggggtt gcctttcatt cagttattca 660

tgccattcga ccggaattgg tggacttgga gacagtgaaa ggcagatcca accgagaaaa 720

tttggaggat gctttcacta tcgccgaaac agaactgggg atcccaagac tgctagatcc 780

tgaagacgtt gatgtggata aaccagatga gaaatctatt atgacctatg tagcccagtt 840

tctgaaacat tatcctgaca ttcacaatgc aagcactgat gggcaagagg atgatagaga 900

agacagagta atttttaagg aaatgaaagt ttggatagaa caatttgaga gagatttgac 960

aagagcacag atggtggaat caaatttaca ggataaatat cagtcattta agcacttcag 1020

agttcaatat gaaatgaaga ggaaacagat tgaacattta atacaaccat tacacagaga 1080

cggtaaattg tcacttgacc aagcattggt aaaacaatct tgggatagag tgacctccag 1140

gctctttgac tggcatatac agcttgataa atctcttcct gcacctctgg gcaccagggc 1200

cc 1202

<210> 13
<211> 21
<212> DNA
<213> Homo sapiens

<400> 13
tccatgcaca gaggatcacc g 21

<210> 14
<211> 51
<212> DNA
<213> Homo sapiens

<400> 14
gaaatacttc caggtttccc atcttttgca aattctgtac aaaattttaa g 51

<210> 15
<211> 2461
<212> DNA
<213> Homo sapiens

<400> 15
gtcgacagat ttgacaagag cacagatggt ggaatcaaat ttacaggata aatatcagtc 60

atttaagcac ttcagagttc aatatgaaat gaagaggaaa cagattgaac atttaataca 120

accattacac agagacggta aattgtcact tgaccaagca ttggtaaaac aatcttggga 180

tagagtgacc tccaggctct ttgactggca tatacagctt gataaatctc ttcctgcacc 240

tctgggcacc ataggtgcct ggctgtacag agcggaggtg gccctgagag aggaaataac 300

cgttcaacag gtccacgagg aaacagcaaa cacgatacaa cggaaacttg agcaacataa 360
ggatctgctt caaaacacgg atgcccacaa aagagcattc catgaaatct accggaccag 420
gtctgttaac gggattccag tgccacctga tcaattagag gacatggccg agaggtttca 480
ttttgtttcc tccacatcag agctacacct aatgaaaatg gaatttttag aattaaagta 540
ccgtctgctc tcactgctgg ttcttgcaga gtcaaagctg aagtcttgga tcattaagta 600
cgggaggaga gagtcagtgg agcagcttct acaaaactac gtgtctttta tagaaaatag 660
caagttcttt gaacaatatg aggtgacata ccagatcttg aaacagacag ctgagatgta 720
tgtcaaagca gatggttcag tggaagaagc tgagaatgtg atgaaattca tgaatgaaac 780
caccgctcag tggaggaatc tctcagtaga agtgaggagt gtgaggagca tgctggaaga 840
agtgatctct aactgggatc gctatggcaa tacagtggct agtctgcaag cctggctaga 900
ggatgctgaa aaaatgctca atcaatcaga aaatgccaaa aaggattttt ttcgaaattt 960
acctcattgg attcagcagc atactgccat gaacgatgct ggcaattttc taattgaaac 1020
ctgtgatgag atggtttccc gtgacctgaa gcagcaatta ctgttgctaa atgggcggtg 1080
gagggagttg tttatggaag tcaagcaata tgctcaagct gatgagatgg acagaatgaa 1140
gaaggaatac acagactgtg ttgttaccct gtctgctttt gcaacggaag cccataagaa 1200
actttctgaa cccttagaag tctcttttat gaatgtcaag ctattaattc aagacttgga 1260
ggatattgag cagagggtgc ctgtgatgga tgcccaatac aagataatta caaagacagc 1320
acacctcatt gccaaagaaa gcccccaaga agaaggaaaa gaaatgtttg cgaccatgtc 1380
aaagctcaaa gagcagctaa ccaaggtcaa agaatgttac tccccactcc tttatgagtc 1440
tcagcagctg ttgattccgt tggaggaatt agaaaagcag atgacgtcct tttatgactc 1500
acttgggaaa atcaatgaaa ttatcacagt tcttgagcgt gaggcacaat cgagtgccct 1560
ttttaaacaa aaacatcagg aactgttagc ttgtcaagaa aactgtaaga aaaccttgac 1620
acttattgag aaaggcagtc aaagtgttca aaagtttgtg accttgagca acgtgttaaa 1680
gcattttgat cagacgaggc tacaaagaca gattgcagat attcatgttg cttttcagag 1740
tatggtaaag aaaactggag attggaagaa gcatgtggaa accaacagtc gcttgatgaa 1800
gaagtttgag gagtctcgag cagagttgga gaaggtactg cggattgctc aggagggcct 1860
ggaggaaaag ggggatccag aggagctcct gcggagacac actgagtttt tcagtcagct 1920
ggatcagagg gtgctcaatg ctttcctgaa agcttgtgat gaactcaccg acatccttcc 1980
agagcaggag cagcaggggc tgcaggaagc tgttcgaaag ctccacaaac aatggaagga 2040
tcttcaagga gaagcccctt atcatttgct tcatctgaag attgatgtgg agaagaatag 2100
gttcttagcc tctgcagaag aatgcagaac tgagctggat cgagagacca agctgatgcc 2160
ccaggaaggc agtgaaaaga taattaaaga gcacagggtt ttcttcagtg acaaaggtcc 2220
tcatcatctc tgtgagaaaa ggttacagct catcgaggaa ctctgtgtga aactcccagt 2280
gcgggaccca gtaaggacac cacctggaac ctgtcacgtg actctcaaag agctcagagc 2340

tgccattgac agcacctaca ggaagctcat ggaagaccca gacaagtgga aggactacac 2400
tagcagattc tccgagttct catcttggat atctacaaat gagacacaat taaaggggcc 2460
c 2461

<210> 16
<211> 2551
<212> DNA
<213> Homo sapiens

<400> 16
gtcgaccgag gaactctgtg tgaaactccc agtgggggac ccagtaaggg acacacctgg 60
aacctgtcac gtgactctca aagagctcag agctgccatt gacagcacct acaggaagct 120
catggaagac ccagacaagt ggaaggacta cactagcaga ttctctgagt tctcatcttg 180
gatatctaca aatgagacac aattaaaggg gatcaagggt gaggccatcg atactgccaa 240
ccacggagag gttaaacgtg ccgttgaaga gatcagaaat ggtgttacca aaaggggtga 300
gaccctcagc tggctgaaat ccaggctgaa agttttgaca gaagtttctt ctgagaatga 360
agcccaaaag cagggagatg agctggcaaa attatccagc tctttcaagg ctcttgtgac 420
gctgctgtca gaggttgaaa agatgctaag caattttggg gactgtgtcc agtacaaaga 480
aatagtcaaa aattctctcg aagaattaat ttctggctct aaagaagtcc aggaacaagc 540
tgagaagatc ttggatactg aaaatctgtt tgaagcacag cagttacttc ttcatcacca 600
gcaaaagaca aagcggatcc cagcaaagaa gagagatgtg cagcagcaga tcgcgcaggc 660
gcagcaggga gaaggggggc tgcctgaccg aggccacgag gagctgcgga agctggagag 720
ctcactggat ggcctggagc gcagccggga gaggcaggaa cgccgcatcc aggtcacatt 780
aagaaaatgg gggcgatttg aaacaaacaa agaaacagta gtaagatacc tttttcaaac 840
aggttccagt catgaacgct tcttgagttt tagcagtttg gaaagtttat cttcagaact 900
ggaacaaaca aaggagtttt ctaaacggac agaaagtatt gcagtccagg ctgagaacct 960
tgtaaaggaa gcttcagaga taccgcttgg gccccaaaat aagcagctgc ttcaacagca 1020
ggccaagtca atcaaagaac aagtcaaaaa attagaagac acgcttgaag aagatattaa 1080
aaccatggaa atggtgaaaa ccaagtggga tcattttggc agtaattttg agactctgtc 1140
cgtctggata actgagaaag aaaaagaact caatgccttg gaaacttcgt catctgccat 1200
ggacatgcaa atcagccaaa ttaaggtcac aattcaggaa atagaaagta agctcagcag 1260
cattgtagga ttagaagaag aagcccagtc ttttgctcag tttgttacca ctggagaatc 1320
tgctcgaatt aaagccaagt tgacacaaat aagaagatac ggggaagagc ttcgagagca 1380
tgcacagtgt ctggaaggaa caatcctggg acatttatct cagcagcaaa agtttgaaga 1440
gaaccttaga aagatccagc aatctgtgtc tgaatttgaa gataaacttg ctgttccaat 1500
taaaatatgt tcttcagcta cagaaacata caaagttctt caagaacata tggatctctg 1560
ccaggccctg gagtcactga gcagcgcgat cactgccttc tcagccagtg ccaggaaggt 1620
tgtgaacaga gattcctgtg ttcaggaggc tgcggctcta cagcagcaat acgaggacat 1680

cctaaggagg gcgaaggaga gacagacggc gctggagaat ctgctggccc actggcagag 1740

gctagagaaa gaactatcat cctttttgac ctggttagag cggggtgaag ctaaagccag 1800

ttccccagaa atggacattt ctgcagacag agtcaaagtg gaaggtgaac ttcagttaat 1860

acaggcactg caaaatgaag ttgtatccca ggcctcattc tatagcaaac ttttgcaatt 1920

gaaggaatca ttgttctcag tagcctccaa agatgatgtg aaaatgatga aactacattt 1980

ggagcagttg gatgagagat ggagagattt accacagatc attaacaaaa ggattaattt 2040

tcttcagtct gtggttgctg aacaccagca atttgatgag ctgctgcttt ccttttctgt 2100

ctggattaaa ctgtttctta gtgaattaca aactacctct gagattagca taatggacca 2160

tcaagtagcc cttactcggc ataaggacca cgcagcagaa gtagagagca aaaagggcga 2220

attgcagagt ctgcagggtc acttagcaaa gttgggttct ctgggccgtg ctgaggacct 2280

ccacctcctg cagggaaagg ctgaggactg cttccagctg tttgaggagg ccagccgggt 2340

tgtggagagg cggcagcttg ccctgtccca tttggcagaa ttcctccaga gccatgcctc 2400

tctgtccggc attctccgcc agctgaggca aacagtggaa gcaaccaaca gtatgaataa 2460

gaacgagtct gatttgatag aaaaggacct caatgatgct cttcaaaatg ctaaagcatt 2520

agaatctgct gccgtcagtc tggatgggcc c 2551

<210> 17
<211> 1120
<212> DNA
<213> Homo sapiens

<400> 17
ggagggcgaa ggagagacag acggcgctgg agaatctgct ggcccactgg cagaggctag 60

agaaagaact atcatccttt ttgacctggt tagagcgggg tgaagctaaa gccagttccc 120

cagaaatgga catttctgca gacagagtca aagtggaagg tgaacttcag ttaatacagg 180

cactgcaaaa tgaagttgta tcccaggcct cattctatag caaacttttg caattgaagg 240

aatcattgtt ctcagtagcc tccaaagatg atgtgaaaat gatgaaacta catttggagc 300

agttggatga gagatggaga gatttaccac agatcattaa caaaaggatt aattttcttc 360

agtctgtggt tgctgaacac cagcaatttg atgagctgct gctttccttt tctgtctgga 420

ttaaactgtt tctcagtgaa ttacaaacta cctctgagat tagcataatg gaccatcaag 480

tagcccttac tcggcataag gaccacgcag cagaagtaga gagcaaaaag ggcgaattgc 540

agagtctgca gggtcactta gcaaagttgg gttctctggg ccgtgctgag gacctccacc 600

tcctgcaggg aaaggctgag gactgcttcc agctgtttga ggaggccagc caggttgtgg 660

agaggcggca gcttgccctg tcccatttgg cagaattcct ccagagccat gcctctctgt 720

ccggcattct ccgccagctg aggcaaacag tggaagcaac caacagtatg aataagaacg 780

agtctgattt gatagaaaag gacctcaatg atgctcttca aaatgctaaa gcattagaat 840

ctgctgccgt cagtctggat ggtattcttt ccaaagccca ataccatctg aaaatcggga 900

gctctgagca aaggacttcc tgcagagcca cggctgatca gctctgtgga gaggtagaga 960

ggatccagaa ccttctggga accaagcaga gtgaggcaga tgctctggca gtgttgaaaa 1020

aagcattcca agaccagaaa gaggagcttc tgaaaagcat tgaggacatt gaagaaagga 1080

ctgacaaaga gcgattgaaa gaacctaccc gccaagctct 1120

<210> 18
<211> 1601
<212> DNA
<213> Homo Sapiens

<400> 18
cattctttcc aaagcccaat accatctgaa aatcgggagc tctgagcaaa ggacttcctg 60

cagagccacg gctgatcagc tctgtggaga ggtagagagg atccagaacc ttctgggaac 120

caagcagagt gaggcagatg ctctggcagt gttgaaaaaa gcattccaag accagaaaga 180

ggagcttctg aaaagcattg aggacattga agaaaggact gacaaagagc gattgaaaga 240

acctacccgc caagctcttc agcagaggtt aagagtgttt aatcagctag aagatgaatt 300

gaattctcac gagcatgaac tatgttggtt gaaagacaaa gccaagcaaa ttgcccagaa 360

agatgtagct tttgcacctg aagttgacag ggagataaac cgcttagagg tcacctggga 420

tgataccaaa agactaattc atgaaaatca gggtcagtgc tgtggactta ttgacttaat 480

gagagaatat cagaacctga aatcagctgt atctaaagtc ttagaaaatg ccagcagtgt 540

gattgtaacc agaactacca taaaagatca ggaggatctt aaatgggctt tttccaagca 600

tgaaactgcc aagaacaaaa tgaattacaa acagaaagac ttggataact ttaccagcaa 660

aggaaaacac ttgttatctg agctgaagaa aattcacagt agtgatttca gcttggtgaa 720

aacagacatg gagagcaccg tggacaaatg gctggatgta tcagagaaac ttgaagaaaa 780

catggatagg ctgagagtaa gcctgtccat ttggggtgat gtactgtcaa ctagagatga 840

gattgaggga tggtcaaaca actgcgttcc acagatggca gaaaacatca gcaacctgga 900

taaccacctc agagctgaag aactgcttaa agaatttgag tctgaagtta aaaacaaagc 960

attgagattg gaagaactgc attccaaagt taatgatctg aaagaattaa ctaaaaatct 1020

agaaacaccg ccagaccttc agtttataga agcagactta atgcagaaac tggagcatgc 1080

caaagaaata actgaagtag caaaaggaac cctgaaggat ttcacggctc aaagtacaca 1140

agtggagaag tttattaatg acataacaac atggttcaca aaagtggaag aatcgttgat 1200

gaactgtgcc caaaatgaga cttgtgaagc attgaaaaaa gtcaaggata tacaaaaaga 1260

acttcaaagt caacaaagca acatcagctc tacccaagaa aatctcaata gcttgtgccg 1320

caagtaccac tcagctgagt tggagagcct gggccgtgca atgactggtc tgataaagaa 1380

acatgaagcc gtgagccagt tgtgctccaa aacccaggcc agcctgcagg aatctctgga 1440

aaaacacttc agtgagtcta tgcaggaatt ccaagaatgg tttttgggag caaaggcagc 1500

agcaaaagaa tcatcagatc gcaccggtga cagcaaagtt ctagaagcaa agctccatga 1560

tcttcagaac attttggact cagtcagtga tgggcagagc a 1601

<210> 19
<211> 1694
<212> DNA
<213> Homo sapiens

<400> 19
gccgtgcaat gcctggtctg ataaagaaac atgaagccgt gagccagttg tgctccaaaa 60

cccaggccag cctgcaggaa tctctggaaa aacacttcag tgagtctatg caggaattcc 120

aagaatggtt tttgggagca aaggcagcag caaaagaatc atcagatcgc accggtgaca 180

gcaaagttct agaagcaaag ctccatgatc ttcagaacat tttggactca gtcagtgatg 240

ggcagagcaa acttgatgca gtgactcaag aaggacaaac tttgtatgca catttgtcta 300

aacaaattgt cagtagcatt caagaacaaa tcacaaaggc caatgaagag tttcaagcat 360

ttctgaaaca atgccttaaa gataagcagg ctcttcaaga ctgtgcttca gaacttggaa 420

gctttgaaga tcagcacaga aaactgaact tatggatcca tgaaatggaa gaaaggttca 480

atacggaaaa cttgggagag agtaaacagc acattcctga gaagaaaaat gaagttcata 540

aagttgaaat gtttttggga gaactgctgg ctgcaagaga gtctcttgat aagctttccc 600

agagagggca gcttctgagt gaagaaggcc acggtgctgg gcaggagggc cgcctgtgtt 660

cccagctcct cacaagccac cagaacctac ttagaatgac caaagagaaa ctccggagct 720

gccaggtggc ccttcaggag cacgaagccc tggaggaagc actgcaaagc atgtggttct 780

gggtgaaggc cattcaggac agactggcct gtgcagagag cactcttggg agcaaagaca 840

ccctggagaa acggctgtca caaatacagg atattctcct gatgaaaggt gaaggggaag 900

ttaagttgaa tatggccatt ggcaaggggg aacaggcctt gagaagtagc aacaaagaag 960

gtcagagggt gattcagact cagttagaga cccttaaaga agtgtgggct gacatcatga 1020

gctcctccgt ccacgctcaa agcactttag agtctgtgat tagccaatgg aatgactatg 1080

tagagaggaa aaaccagttg gagcagtgga tggaatcagt ggatcaaaaa atagaacatc 1140

cctcacaacc acagccaggt ctgaaagaga agttcgtcct gcttgaccac ctccagtcca 1200

tcctgtctga ggcagaagat cacacgagag cccttcaccg tctaattgcg aagtccaggg 1260

agttctacga aaagacagag gatgagtctt tcaaggacac agctcaagag gagctgaaaa 1320

cacagtttaa tgatataatg actgttgcca aggaaaaaat gaggaaagtg gaagagattg 1380

tgaaagatca tctaatgtat ttagatgcgg tccacgagtt cacagattgg ctccattcag 1440

caaaggaaga acttcaccgg tggtcagata tgtctggaga ttcatcagcc acccagaaaa 1500

agttatcaaa aattaaggag ctgatagatt ccagagagat tggtgcaagc cgtctcagca 1560

gagtggagtc gctggctccc gaagtgaaac agaacacaac tgccagtggg tgtgagctca 1620

tgcacacgga gatgcaggcc ctgcgtgccg actggaagca gtgggaagac agtgtattcc 1680

aaacgcagag ctgt 1694

<210> 20

<211>  1505
<212>  DNA
<213>  Homo sapiens

<400>  20
gtcgactctc ctcacaagcc accagaacct acttagaatg accaaagaga aactccggag      60

ctgccaggtg gcccttcagg agcacgaagc cctggaggaa gcactgcaaa gcatgtggtt     120

ctgggtgaag gccattcagg acagactggc ctgtgcagag agcactcttg ggagcaaaga     180

caccctggag aaacggctgt cacaaataca ggatattctc ctgatgaaag gtggagggga     240

agttaagttg aatatggcca ttggcaaggg ggaacaggcc ttgagaagta gcaacaaaga     300

aggtcagagg gtgattcaga ctcagttaga gacccttaaa gaagtgtggg ctgacatcat     360

gagctcctcc gtccacgctc aaagcacttt agagtctgtg attagccaat ggaatgacta     420

tgtagagagg aaaaaccagt tggagcagtg gatggaatca gtggatcaaa aaatagaaca     480

tcccttacaa ccacagccag gtctgaaaga gaagttcgtc ctgcttgacc acctccagtc     540

catcctgtct gaggcagaag atcacacgag agcccttcac cgtctaattg tgaagtccag     600

ggagctctac gaaaagacag aggatgagtc tttcaaggac acagctcaag aggagctgaa     660

aacacagttt aatgatataa tgactgttgc caaggaaaaa atgaggaaag tggaagagat     720

tgtgaaagat catctaatgt atttagatgc ggtccacgag ttcacagatt ggctccattc     780

agcaaaggaa gaacttcacc ggtggtcaga tatgtctgga gattcatcag ccacccagaa     840

aaagttatca aaaattaagg agctgataga ttccagagag attggtgcaa gccgtctcag     900

cagagtggag tcgctggctc ccgaagtgaa acagaacaca actgccagtg ggtgtgagct     960

catgcacacg gagatgcagg ccctgcgtgc cgactggaag cagtgggaag acagtgtatt    1020

ccaaacgcag agctgtttgg agaacctggt cagccagatg gccctttcgg agcaggaatt    1080

ctcaggccaa gtggctcaac tggagcaggc cctggaacag ttcagtgccc ttctgaaaac    1140

ctgggctcag cagttaaccc tcctggaagg caagaacacg gatgaggaga tagtggaatg    1200

ctggcacaaa ggacaagaga tactggatgc tttgcaaaaa gcagagccta gaacagagga    1260

tctcaagtct cagctgaatg aactttgtcg attttccaga gacctgagta cctacagtgg    1320

aaaagtttct ggcttgatta aagagtataa ttggtgagca tgaaccttat tggtgttcaa    1380

gatattttta tacaaattag aagctagaag tttattttaa ggtgtaattt caaaataata    1440

ttagttcagc attaaatata ttactaagtg aggtaagtgc acattctaag aactggtgac    1500

ccggg                                                                1505

<210>  21
<211>  681
<212>  DNA
<213>  Homo sapiens

<400>  21
acaaggcacc agaacctact tagaatgacc aaagagaaac tccggagctg ccaggtggcc      60

cttcaggagc acgaagccct ggaggaagca ctgcaaagca tgtggttctg ggtgaaggcc     120

attcaggaca gactggcctg tgcagagagc actcttggga gcaaagacac cctggagaaa 180

cggctgtcac aaatacagga tattctcctg atgaaaggtg aaggggaagt taagttgaat 240

atggccattg gcaaggggga acaggccttg agaagtagca acaaagaagg tcagagggtg 300

attcagactc agttagagac ccttaaagaa gtgtgggctg acatcatgag ctcctccgtc 360

cacgctcaaa gcactttaga gtctgtgatt agccaatgga atgactatgt agagaggaaa 420

aaccagttgg agcagtggat ggaatcagtg gatcaaaaaa tagaacatcc cttacaacca 480

cagccaggtc tgaaagagaa gttcgtcctg cttgaccacc tccagtccat cctgtctgag 540

gcagaagatc acacgagagc ccttcaccgt ctaattgcga agtccaggga gctctacgaa 600

aagacagagg atgagtcttt caaggacaca gctcaagagg agctgaaaac acagtttaat 660

gatataatga ctgttgccaa a 681

<210> 22
<211> 24
<212> DNA
<213> Homo sapiens

<400> 22
ctggagaaac ggctgtcaca aata 24

<210> 23
<211> 28
<212> DNA
<213> Homo sapiens

<400> 23
tagccaatgg aatgactatg tagagagg 28

<210> 24
<211> 1093
<212> DNA
<213> Homo sapiens

<400> 24
atgtatttag atgcggtcca cgagttcaca gattggctcc attcagcaaa ggaagaactt 60

caccggtggt cagatatgtc tggagattca tcagccaccc agaaaaagtt atcaaaaatt 120

aaggagctga tagattccag agagattggt gcaagccgtc tcagcagagt ggagtcgctg 180

gctcccgaag tgaaacagaa cacaactgcc agtgggtgtg agctcatgca cacggagatg 240

caggccctgc gtgccgactg gaagcagtgg gaagacagtg tattccaaac gcagagctgt 300

ttggagaacc tggtcagcca gatggccctt tcggagcagg aattctcagg ccaagtggct 360

caactggagc aggccctgga acagttcagt gcccttctga aaacctgggc tcagcagtta 420

accctcctgg aaggcaagaa cacggatgag gagatagtgg aatgctggca caaaggacaa 480

gagatactgg atgctttgca aaaagcagag cctagaacag aggatctcaa gtctcagctg 540

aatgaacttt gtcgattttc cagagacctg agtacctaca gtggaaaagt ttctggcttg 600

attaaagagt ataattggtg agcatgaacc ttattggtgt tcaagatatt tttatacaaa 660

ttagaagcta gaagtttatt ttaaggtgta atttcaaaat aatattagtt cagcattaaa 720

tatattacta agtgaggtaa gtgcacattc taagaactgg tgattctatc aaacatgatt 780

ttcatcaact ttatcgcttt ttcctgccat taaatttttg cgtcaccttt tcagtctttg 840

tttgcaagca tccaagggct gccagaataa agaacagatt ttacagcaaa gatttcgaaa 900

ggccttcagg gatttccagc agtggttggt taatgcaaaa atcactaccg ccaagtgttt 960

tgatatacct caaaatataa gtgaagtttc aactagtctt cagaaaatac aggtaagggt 1020

gttatcaatt aattctagta gcagtgctga tgtgaactaa agttaacctc taaatcaagg 1080

gtccccagtc gac 1093

<210> 25
<211> 357
<212> DNA
<213> Homo sapiens

<400> 25
gagctgatag attccagaga gattggtgca agccgtctca gcagagtgga gtcgctggct 60

cccgaagtga aacagaacac aactgccagt gggtgtgagc tcatgcacac ggagatgcag 120

gccctgcgtg ccgactggaa gcagtgggaa gacagtgtat tccaaacgca gagctgtttg 180

gagaacctgg tcagccagat ggccctttcg gagcaggaat tctcaggcca agtggctcaa 240

ctggagcagg ccctggaaca gttcagtgcc cttctgaaaa cctgggctca gcagttaacc 300

ctcctggaag gcaagaacac ggatgaggag atagtggaat gctggcacaa aggacaa 357

<210> 26
<211> 137
<212> DNA
<213> Homo sapiens

<400> 26
gagatactgg atgctttgca aaaagcagag cctagaacag aggatctcaa gtctcagctg 60

aatgaacttt gtcgattttc cagagacctg agtacctaca gtggaaaagt ttctggcttg 120

attaaagagt ataattg 137

<210> 27
<211> 878
<212> DNA
<213> Homo sapiens

<400> 27
ggagatagtg gaatgctggc acaaaggaca agagatactg gatgctttgc aaaaagcaga 60

gcctagaaca gaggatctca agtctcagct gaatgaactt tgtcgatttt ccagagacct 120

gagtacctac agtggaaaag tttctggctt gattaaagag tataattggt gagcatgaac 180

cttattggtg ttcaagatat ttttatacaa attagaagct agaagtttat tttaaggtgt 240

aatttcaaaa taatattagt tcagcattaa atatattact aagtgaggta agtgcacatt 300

ctaagaactg gtgattctat caaacatgat tttcatcaac tttatcgctt tttcctgcca 360

ttaaattttt gcgtcacctt ttcagtcttt gtttgcaagc atccaagggc tgccagaata 420

aagaacagat tttacagcaa agatttcgaa aggccttcag ggatttccag cagtggttgg 480

ttaatgcaaa aatcactacc gccaagtgtt ttgatatacc tcaaaatata agtgaagttt 540

caactagtct tcagaaaata caggtaaggg tgttatcaat taattctagt agcagtgctg 600

atgtgaacta aagttaacct ctaaatcaag ggtccccatc tcctgggccg cggactggta 660

caggtccctg gcctgttagg aaccaggccg cacagcagga ggtgagcagc aggccaatga 720

gcgttactgc ctgagctcca cctcctgtca gatcagcggt agcatcagat tctcatagga 780

gcctgaaccc cattgtgaac tgtgcacaca gaggaactag gctgtgtgct ccttctaaga 840

atctaactaa tgcctgatga tcgaggtaga acagtttc 878

<210> 28
<211> 723
<212> DNA
<213> Homo sapiens

<400> 28
gagatactgg atgctttgca aaaagcagag cctagaacag aggatctcaa gtctcagctg 60

aatgaacttt gtcgattttc cagagacctg agtacctaca gtggaaaagt ttctggcttg 120

attaaagagt ataattggtg agcatgaacc ttattggtgt tcaagatatt tttatacaaa 180

ttagaagcta gaagtttatt ttaaggtgta atttcaaaat aatattagtt cagcattaaa 240

tatattacta agtgaggtaa gtgcacattc taagaactgg tgattctatc aaacatgatt 300

ttcatcaact ttatcgcttt ttcctgccat taaatttttg cgtcaccttt tcagtctttg 360

tttgcaagca tccaagggct gccagaataa agaacagatt ttacagcaaa gatttcgaaa 420

ggccttcagg gatttccagc agtggttggt taatgcaaaa atcactaccg ccaagtgttt 480

tgatatacct caaaatataa gtgaagtttc aactagtctt cagaaaatac aggtaagggt 540

gttatcaatt aattctagta gcagtgctga tgtgaactaa agttaacctc taaatcaagg 600

gtccccatct cctgggccgc ggactggtac aggtccctgg cctgttagga accaggccgc 660

acagcaggag gtgagcagca ggccaatgag cgttactgcc tgagctccac ctcctgtcag 720

atc 723

<210> 29
<211> 178
<212> DNA
<213> Homo sapiens

<400> 29
tctttgtttg caagcatcca agggctgcca gaataaagaa cagattttac agcaaagatt 60

tcgaaaggcc ttcagggatt tccagcagtg gttggttaat gcaaaaatca ctaccgccaa 120

gtgttttgat atacctcaaa atataagtga agtttcaact agtcttcaga aaatacag 178

<210> 30
<211> 3032
<212> PRT
<213> Homo sapiens

<400> 30

Met Ala Thr Ser Arg Gly Ala Ser Arg Cys Pro Arg Asp Ile Ala Asn
1               5                   10                  15

Val Met Gln Arg Leu Gln Asp Glu Gln Glu Ile Val Gln Lys Arg Thr
            20                  25                  30

Phe Thr Lys Trp Ile Asn Ser His Leu Ala Lys Arg Lys Pro Pro Met
        35                  40                  45

Val Val Asp Asp Leu Phe Glu Asp Met Lys Asp Gly Val Lys Leu Leu
    50                  55                  60

Ala Leu Leu Glu Val Leu Ser Gly Gln Lys Leu Pro Cys Glu Gln Gly
65                  70                  75                  80

Arg Arg Met Lys Arg Ile His Ala Val Ala Asn Ile Gly Thr Ala Leu
                85                  90                  95

Lys Phe Leu Glu Gly Arg Lys Ile Lys Leu Val Asn Ile Asn Ser Thr
            100                 105                 110

Asp Ile Ala Asp Gly Arg Pro Ser Ile Val Leu Gly Leu Met Trp Thr
        115                 120                 125

Ile Ile Leu Tyr Phe Gln Ile Glu Glu Leu Thr Ser Asn Leu Pro Gln
    130                 135                 140

Leu Gln Ser Leu Ser Ser Ser Ala Ser Ser Val Asp Ser Ile Val Ser
145                 150                 155                 160

Ser Glu Thr Pro Ser Pro Pro Ser Lys Arg Lys Val Thr Thr Lys Ile
                165                 170                 175

Gln Gly Asn Ala Lys Lys Ala Leu Leu Lys Trp Val Gln Tyr Thr Ala
            180                 185                 190

Gly Lys Gln Thr Gly Ile Glu Val Lys Asp Phe Gly Lys Ser Trp Arg
        195                 200                 205

Ser Gly Val Ala Phe His Ser Val Ile His Ala Ile Arg Pro Glu Leu
    210                 215                 220

Val Asp Leu Glu Thr Val Lys Gly Arg Ser Asn Arg Glu Asn Leu Glu
225                 230                 235                 240

Asp Ala Phe Thr Ile Ala Glu Thr Glu Leu Gly Ile Pro Arg Leu Leu
                245                 250                 255

Asp Pro Glu Asp Val Asp Val Asp Lys Pro Asp Glu Lys Ser Ile Met
            260                 265                 270

Thr Tyr Val Ala Gln Phe Leu Lys His Tyr Pro Asp Ile His Asn Ala
        275                 280                 285

Ser Thr Asp Gly Gln Glu Asp Asp Arg Glu Asp Arg Val Ile Phe Lys
    290                 295                 300

Glu Met Lys Val Trp Ile Glu Gln Phe Glu Arg Asp Leu Thr Arg Ala
305                 310                 315                 320

Gln Met Val Glu Ser Asn Leu Gln Asp Lys Tyr Gln Ser Phe Lys His
                325                 330                 335

Phe Arg Val Gln Tyr Glu Met Lys Arg Lys Gln Ile Glu His Leu Ile
            340                 345                 350

Gln Pro Leu His Arg Asp Gly Lys Leu Ser Leu Asp Gln Ala Leu Val
        355                 360                 365

Lys Gln Ser Trp Asp Arg Val Thr Ser Arg Leu Phe Asp Trp His Ile
    370                 375                 380

Gln Leu Asp Lys Ser Leu Pro Ala Pro Leu Gly Thr Ile Gly Ala Trp
385                 390                 395                 400

Leu Tyr Arg Ala Glu Val Ala Leu Arg Glu Glu Ile Thr Val Gln Gln
                405                 410                 415

Val His Glu Glu Thr Ala Asn Thr Ile Gln Arg Lys Leu Glu Gln His
            420                 425                 430

Lys Asp Leu Leu Gln Asn Thr Asp Ala His Lys Arg Ala Phe His Glu
        435                 440                 445

Ile Tyr Arg Thr Arg Ser Val Asn Gly Ile Pro Val Pro Pro Asp Gln
    450                 455                 460

Leu Glu Asp Met Ala Glu Arg Phe His Phe Val Ser Ser Thr Ser Glu
465                 470                 475                 480

Leu His Leu Met Lys Met Glu Phe Leu Glu Leu Lys Tyr Arg Leu Leu
                485                 490                 495

Ser Leu Leu Val Leu Ala Glu Ser Lys Leu Lys Ser Trp Ile Ile Lys
            500                 505                 510

Tyr Gly Arg Arg Glu Ser Val Glu Gln Leu Leu Gln Asn Tyr Val Ser
        515                 520                 525

Phe Ile Glu Asn Ser Lys Phe Phe Glu Gln Tyr Glu Val Thr Tyr Gln
    530                 535                 540

Ile Leu Lys Gln Thr Ala Glu Met Tyr Val Lys Ala Asp Gly Ser Val
545                 550                 555                 560

Glu Glu Ala Glu Asn Val Met Lys Phe Met Asn Glu Thr Thr Ala Gln
                565                 570                 575

Trp Arg Asn Leu Ser Val Glu Val Arg Ser Val Arg Ser Met Leu Glu
            580                 585                 590

Glu Val Ile Ser Asn Trp Asp Arg Tyr Gly Asn Thr Val Ala Ser Leu
        595                 600                 605

Gln Ala Trp Leu Glu Asp Ala Glu Lys Met Leu Asn Gln Ser Glu Asn
    610                 615                 620

Ala Lys Lys Asp Phe Phe Arg Asn Leu Pro His Trp Ile Gln Gln His
625                 630                 635                 640

Thr Ala Met Asn Asp Ala Gly Asn Phe Leu Ile Glu Thr Cys Asp Glu
                645                 650                 655

Met Val Ser Arg Asp Leu Lys Gln Gln Leu Leu Leu Leu Asn Gly Arg
            660                 665                 670

Trp Arg Glu Leu Phe Met Glu Val Lys Gln Tyr Ala Gln Ala Asp Glu
        675                 680                 685

Met Asp Arg Met Lys Lys Glu Tyr Thr Asp Cys Val Val Thr Leu Ser
    690                 695                 700

Ala Phe Ala Thr Glu Ala His Lys Lys Leu Ser Glu Pro Leu Glu Val
705                 710                 715                 720

Ser Phe Met Asn Val Lys Leu Leu Ile Gln Asp Leu Glu Asp Ile Glu
                725                 730                 735

Gln Arg Val Pro Val Met Asp Ala Gln Tyr Lys Ile Ile Thr Lys Thr
            740                 745                 750

Ala His Leu Ile Thr Lys Glu Ser Pro Gln Glu Glu Gly Lys Glu Met
        755                 760                 765

Phe Ala Thr Met Ser Lys Leu Lys Glu Gln Leu Thr Lys Val Lys Glu
    770                 775                 780

Cys Tyr Ser Pro Leu Leu Tyr Glu Ser Gln Gln Leu Leu Ile Pro Leu
785                 790                 795                 800

Glu Glu Leu Glu Lys Gln Met Thr Ser Phe Tyr Asp Ser Leu Gly Lys
                805                 810                 815

Ile Asn Glu Ile Ile Thr Val Leu Glu Arg Glu Ala Gln Ser Ser Ala
            820                 825                 830

Leu Phe Lys Gln Lys His Gln Glu Leu Leu Ala Cys Gln Glu Asn Cys
        835                 840                 845

Lys Lys Thr Leu Thr Leu Ile Glu Lys Gly Ser Gln Ser Val Gln Lys
    850                 855                 860

Phe Val Thr Leu Ser Asn Val Leu Lys His Phe Asp Gln Thr Arg Leu
865                 870                 875                 880

Gln Arg Gln Ile Ala Asp Ile His Val Ala Phe Gln Ser Met Val Lys
                885                 890                 895

Lys Thr Gly Asp Trp Lys Lys His Val Glu Thr Asn Ser Arg Leu Met
            900                 905                 910

Lys Lys Phe Glu Glu Ser Arg Ala Glu Leu Glu Lys Val Leu Arg Ile
        915                 920                 925

Ala Gln Glu Gly Leu Glu Glu Lys Gly Asp Pro Glu Glu Leu Leu Arg
    930                 935                 940

Arg His Thr Glu Phe Phe Ser Gln Leu Asp Gln Arg Val Leu Asn Ala
945                 950                 955                 960

Phe Leu Lys Ala Cys Asp Glu Leu Thr Asp Ile Leu Pro Glu Gln Glu
                965                 970                 975

Gln Gln Gly Leu Gln Glu Ala Val Arg Lys Leu His Lys Gln Trp Lys
            980                 985                 990

Asp Leu Gln Gly Glu Ala Pro Tyr  His Leu Leu His Leu  Lys Ile Asp
        995                 1000                1005

Val Glu  Lys Asn Arg Phe Leu  Ala Ser Val Glu Glu  Cys Arg Thr
    1010                1015                1020

Glu Leu  Asp Arg Glu Thr Lys  Leu Met Pro Gln Glu  Gly Ser Glu
    1025                1030                1035

Lys Ile  Ile Lys Glu His Arg  Val Phe Phe Ser Asp  Lys Gly Pro
    1040                1045                1050

His His  Leu Cys Glu Lys Arg  Leu Gln Leu Ile Glu  Glu Leu Cys
    1055                1060                1065

Val Lys  Leu Pro Val Arg Asp  Pro Val Arg Asp Thr  Pro Gly Thr
    1070                1075                1080

Cys His Val Thr Leu Lys Glu Leu Arg Ala Ala Ile Asp Ser Thr
    1085                1090                1095

Tyr Arg Lys Leu Met Glu Asp Pro Asp Lys Trp Lys Asp Tyr Thr
    1100                1105                1110

Ser Arg Phe Ser Glu Phe Ser Ser Trp Ile Ser Thr Asn Glu Thr
    1115                1120                1125

Gln Leu Lys Gly Ile Lys Gly Glu Ala Ile Asp Thr Ala Asn His
    1130                1135                1140

Gly Glu Val Lys Arg Ala Val Glu Glu Ile Arg Asn Gly Val Thr
    1145                1150                1155

Lys Arg Gly Glu Thr Leu Ser Trp Leu Lys Ser Arg Leu Lys Val
    1160                1165                1170

Leu Thr Glu Val Ser Ser Glu Asn Glu Ala Gln Lys Gln Gly Asp
    1175                1180                1185

Glu Leu Ala Lys Leu Ser Ser Ser Phe Lys Ala Leu Val Thr Leu
    1190                1195                1200

Leu Ser Glu Val Glu Lys Met Leu Ser Asn Phe Gly Asp Cys Val
    1205                1210                1215

Gln Tyr Lys Glu Ile Val Lys Asn Ser Leu Glu Glu Leu Ile Ser
    1220                1225                1230

Gly Ser Lys Glu Val Gln Glu Gln Ala Glu Lys Ile Leu Asp Thr
    1235                1240                1245

Glu Asn Leu Phe Glu Ala Gln Gln Leu Leu Leu His His Gln Gln
    1250                1255                1260

Lys Thr Lys Arg Ile Ser Ala Lys Lys Arg Asp Val Gln Gln Gln
    1265                1270                1275

Ile Ala Gln Ala Gln Gln Gly Glu Gly Gly Leu Pro Asp Arg Gly
    1280                1285                1290

His Glu Glu Leu Arg Lys Leu Glu Ser Thr Leu Asp Gly Leu Glu
    1295                1300                1305

Arg Ser Arg Glu Arg Gln Glu Arg Arg Ile Gln Val Thr Leu Arg
    1310                1315                1320

Lys Trp Glu Arg Phe Glu Thr Asn Lys Glu Thr Val Val Arg Tyr
    1325                1330                1335

Leu Phe Gln Thr Gly Ser Ser His Glu Arg Phe Leu Ser Phe Ser
    1340                1345                1350

Ser Leu Glu Ser Leu Ser Ser Glu Leu Glu Gln Thr Lys Glu Phe
    1355                1360                1365

Ser Lys Arg Thr Glu Ser Ile Ala Val Gln Ala Glu Asn Leu Val
    1370                1375                1380

Lys Glu Ala Ser Glu Ile Pro Leu Gly Pro Gln Asn Lys Gln Leu
    1385                1390                1395

Leu Gln Gln Gln Ala Lys Ser Ile Lys Glu Gln Val Lys Lys Leu
    1400                1405                1410

Glu Asp Thr Leu Glu Glu Asp Ile Lys Thr Met Glu Met Val Lys
    1415                1420                1425

Thr Lys Trp Asp His Phe Gly Ser Asn Phe Glu Thr Leu Ser Val
    1430                1435                1440

Trp Ile Thr Glu Lys Glu Lys Glu Leu Asn Ala Leu Glu Thr Ser
    1445                1450                1455

Ser Ser Ala Met Asp Met Gln Ile Ser Gln Ile Lys Val Thr Ile
    1460                1465                1470

Gln Glu Ile Glu Ser Lys Leu Ser Ser Ile Val Gly Leu Glu Glu
    1475                1480                1485

Glu Ala Gln Ser Phe Ala Gln Phe Val Thr Thr Gly Glu Ser Ala
    1490                1495                1500

Arg Ile Lys Ala Lys Leu Thr Gln Ile Arg Arg Tyr Gly Glu Glu
    1505                1510                1515

Leu Arg Glu His Ala Gln Cys Leu Glu Gly Thr Ile Leu Gly His
    1520                1525                1530

Leu Ser Gln Gln Gln Lys Phe Glu Glu Asn Leu Arg Lys Ile Gln
    1535                1540                1545

Gln Ser Val Ser Glu Phe Glu Asp Lys Leu Ala Val Pro Ile Lys
    1550                1555                1560

Ile Cys Ser Ser Ala Thr Glu Thr Tyr Lys Val Leu Gln Glu His
    1565                1570                1575

Met Asp Leu Cys Gln Ala Leu Glu Ser Leu Ser Ser Ala Ile Thr
    1580                1585                1590

Ala Phe Ser Ala Ser Ala Arg Lys Val Val Asn Arg Asp Ser Cys
    1595                1600                1605

Val Gln Glu Ala Ala Ala Leu Gln Gln Gln Tyr Glu Asp Ile Leu
    1610                1615                1620

Arg Arg Ala Lys Glu Arg Gln Thr Ala Leu Glu Asn Leu Leu Ala
    1625                1630                1635

His Trp Gln Arg Leu Glu Lys Glu Leu Ser Ser Phe Leu Thr Trp
    1640                1645                1650

Leu Glu Arg Gly Glu Ala Lys Ala Ser Ser Pro Glu Met Asp Ile
    1655                1660                1665

Ser Ala Asp Arg Val Lys Val Glu Gly Glu Leu Gln Leu Ile Gln
    1670                1675                1680

Ala Leu Gln Asn Glu Val Val Ser Gln Ala Ser Phe Tyr Ser Lys
    1685                1690                1695

Leu Leu Gln Leu Lys Glu Ser Leu Phe Ser Val Ala Ser Lys Asp
    1700                1705                1710

Asp Val Lys Met Met Lys Leu His Leu Glu Gln Leu Asp Glu Arg
    1715                1720                1725

Trp Arg Asp Leu Pro Gln Ile Ile Asn Lys Arg Ile Asn Phe Leu
    1730                1735                1740

Gln Ser Val Val Ala Glu His Gln Gln Phe Asp Glu Leu Leu Leu
    1745                1750                1755

Ser Phe Ser Val Trp Ile Lys Leu Phe Leu Ser Glu Leu Gln Thr
    1760                1765                1770

Thr Ser Glu Ile Ser Ile Met Asp His Gln Val Ala Leu Thr Arg
    1775                1780                1785

His Lys Asp His Ala Ala Glu Val Glu Ser Lys Lys Gly Glu Leu
    1790                1795                1800

Gln Ser Leu Gln Gly His Leu Ala Lys Leu Gly Ser Leu Gly Arg
    1805                1810                1815

Ala Glu Asp Leu His Leu Leu Gln Gly Lys Ala Glu Asp Cys Phe
    1820                1825                1830

Gln Leu Phe Glu Glu Ala Ser Gln Val Val Glu Arg Arg Gln Leu
    1835                1840                1845

Ala Leu Ser His Leu Ala Glu Phe Leu Gln Ser His Ala Ser Leu
    1850                    1855                    1860

Ser Gly Ile Leu Arg Gln Leu Arg Gln Thr Val Glu Ala Thr Asn
    1865                    1870                    1875

Ser Met Asn Lys Asn Glu Ser Asp Leu Ile Glu Lys Asp Leu Asn
    1880                    1885                    1890

Asp Ala Leu Gln Asn Ala Lys Ala Leu Glu Ser Ala Ala Val Ser
    1895                    1900                    1905

Leu Asp Gly Ile Leu Ser Lys Ala Gln Tyr His Leu Lys Ile Gly
    1910                    1915                    1920

Ser Ser Glu Gln Arg Thr Ser Cys Arg Ala Thr Ala Asp Gln Leu
    1925                    1930                    1935

Cys Gly Glu Val Glu Arg Ile Gln Asn Leu Leu Gly Thr Lys Gln
    1940                    1945                    1950

Ser Glu Ala Asp Ala Leu Ala Val Leu Lys Lys Ala Phe Gln Asp
    1955                    1960                    1965

Gln Lys Glu Glu Leu Leu Lys Ser Ile Glu Asp Ile Glu Glu Arg
    1970                    1975                    1980

Thr Asp Lys Glu Arg Leu Lys Glu Pro Thr Arg Gln Ala Leu Gln
    1985                    1990                    1995

Gln Arg Leu Arg Val Phe Asn Gln Leu Glu Asp Glu Leu Asn Ser
    2000                    2005                    2010

His Glu His Glu Leu Cys Trp Leu Lys Asp Lys Ala Lys Gln Ile
    2015                    2020                    2025

Ala Gln Lys Asp Val Ala Phe Ala Pro Glu Val Asp Arg Glu Ile
    2030                    2035                    2040

Asn Arg Leu Glu Val Thr Trp Asp Asp Thr Lys Arg Leu Ile His
    2045                    2050                    2055

Glu Asn Gln Gly Gln Cys Cys Gly Leu Ile Asp Leu Met Arg Glu
    2060                    2065                    2070

Tyr Gln Asn Leu Lys Ser Ala Val Ser Lys Val Leu Glu Asn Ala
    2075                    2080                    2085

Ser Ser Val Ile Val Thr Arg Thr Thr Ile Lys Asp Gln Glu Asp
    2090                    2095                    2100

Leu Lys Trp Ala Phe Ser Lys His Glu Thr Ala Lys Asn Lys Met
    2105                2110                2115

Asn Tyr Lys Gln Lys Asp Leu Asp Asn Phe Thr Ser Lys Gly Lys
    2120                2125                2130

His Leu Leu Ser Glu Leu Lys Lys Ile His Ser Ser Asp Phe Ser
    2135                2140                2145

Leu Val Lys Thr Asp Met Glu Ser Thr Val Asp Lys Trp Leu Asp
    2150                2155                2160

Val Ser Glu Lys Leu Glu Glu Asn Met Asp Arg Leu Arg Val Ser
    2165                2170                2175

Leu Ser Ile Trp Asp Asp Val Leu Ser Thr Arg Asp Glu Ile Glu
    2180                2185                2190

Gly Trp Ser Asn Asn Cys Val Pro Gln Met Ala Glu Asn Ile Ser
    2195                2200                2205

Asn Leu Asp Asn His Leu Arg Ala Glu Glu Leu Leu Lys Glu Phe
    2210                2215                2220

Glu Ser Glu Val Lys Asn Lys Ala Leu Arg Leu Glu Glu Leu His
    2225                2230                2235

Ser Lys Val Asn Asp Leu Lys Glu Leu Thr Lys Asn Leu Glu Thr
    2240                2245                2250

Pro Pro Asp Leu Gln Phe Ile Glu Ala Asp Leu Met Gln Lys Leu
    2255                2260                2265

Glu His Ala Lys Glu Ile Thr Glu Val Ala Lys Gly Thr Leu Lys
    2270                2275                2280

Asp Phe Thr Ala Gln Ser Thr Gln Val Glu Lys Phe Ile Asn Asp
    2285                2290                2295

Ile Thr Thr Trp Phe Thr Lys Val Glu Glu Ser Leu Met Asn Cys
    2300                2305                2310

Ala Gln Asn Glu Thr Cys Glu Ala Leu Lys Lys Val Lys Asp Ile
    2315                2320                2325

Gln Lys Glu Leu Gln Ser Gln Gln Ser Asn Ile Ser Ser Thr Gln
    2330                2335                2340

Glu Asn Leu Asn Ser Leu Cys Arg Lys Tyr His Ser Ala Glu Leu
    2345                2350                2355

Glu Ser Leu Gly Arg Ala Met Thr Gly Leu Ile Lys Lys His Glu
    2360                    2365                    2370

Ala Val Ser Gln Leu Cys Ser Lys Thr Gln Ala Ser Leu Gln Glu
    2375                    2380                    2385

Ser Leu Glu Lys His Phe Ser Glu Ser Met Gln Glu Phe Gln Glu
    2390                    2395                    2400

Trp Phe Leu Gly Ala Lys Ala Ala Ala Lys Glu Ser Ser Asp Arg
    2405                    2410                    2415

Thr Gly Asp Ser Lys Val Leu Glu Ala Lys Leu His Asp Leu Gln
    2420                    2425                    2430

Asn Ile Leu Asp Ser Val Ser Asp Gly Gln Ser Lys Leu Asp Ala
    2435                    2440                    2445

Val Thr Gln Glu Gly Gln Thr Leu Tyr Ala His Leu Ser Lys Gln
    2450                    2455                    2460

Ile Val Ser Ser Ile Gln Glu Gln Ile Thr Lys Ala Asn Glu Glu
    2465                    2470                    2475

Phe Gln Ala Phe Leu Lys Gln Cys Leu Lys Asp Lys Gln Ala Leu
    2480                    2485                    2490

Gln Asp Cys Ala Ser Glu Leu Gly Ser Phe Glu Asp Gln His Arg
    2495                    2500                    2505

Lys Leu Asn Leu Trp Ile His Glu Met Glu Glu Arg Phe Asn Thr
    2510                    2515                    2520

Glu Asn Leu Gly Glu Ser Lys Gln His Ile Pro Glu Lys Lys Asn
    2525                    2530                    2535

Glu Val His Lys Val Glu Met Phe Leu Gly Glu Leu Leu Ala Ala
    2540                    2545                    2550

Arg Glu Ser Leu Asp Lys Leu Ser Gln Arg Gly Gln Leu Leu Ser
    2555                    2560                    2565

Glu Glu Gly His Gly Ala Gly Gln Glu Gly Arg Leu Cys Ser Gln
    2570                    2575                    2580

Leu Leu Thr Ser His Gln Asn Leu Leu Arg Met Thr Lys Glu Lys
    2585                    2590                    2595

Leu Arg Ser Cys Gln Val Ala Leu Gln Glu His Glu Ala Leu Glu
    2600                    2605                    2610

Glu Ala Leu Gln Ser Met Trp Phe Trp Val Lys Ala Ile Gln Asp
    2615                2620                2625

Arg Leu Ala Cys Ala Glu Ser Thr Leu Gly Ser Lys Asp Thr Leu
    2630                2635                2640

Glu Lys Arg Leu Ser Gln Ile Gln Asp Ile Leu Leu Met Lys Gly
    2645                2650                2655

Glu Gly Glu Val Lys Leu Asn Met Ala Ile Gly Lys Gly Glu Gln
    2660                2665                2670

Ala Leu Arg Ser Ser Asn Lys Glu Gly Gln Arg Val Ile Gln Thr
    2675                2680                2685

Gln Leu Glu Thr Leu Lys Glu Val Trp Ala Asp Ile Met Ser Ser
    2690                2695                2700

Ser Val His Ala Gln Ser Thr Leu Glu Ser Val Ile Ser Gln Trp
    2705                2710                2715

Asn Asp Tyr Val Glu Arg Lys Asn Gln Leu Glu Gln Trp Met Glu
    2720                2725                2730

Ser Val Asp Gln Lys Ile Glu His Pro Leu Gln Pro Gln Pro Gly
    2735                2740                2745

Leu Lys Glu Lys Phe Val Leu Leu Asp His Leu Gln Ser Ile Leu
    2750                2755                2760

Ser Glu Ala Glu Asp His Thr Arg Ala Leu His Arg Leu Ile Ala
    2765                2770                2775

Lys Ser Arg Glu Leu Tyr Glu Lys Thr Glu Asp Glu Ser Phe Lys
    2780                2785                2790

Asp Thr Ala Gln Glu Glu Leu Lys Thr Gln Phe Asn Asp Ile Met
    2795                2800                2805

Thr Val Ala Lys Glu Lys Met Arg Lys Val Glu Glu Ile Val Lys
    2810                2815                2820

Asp His Leu Met Tyr Leu Asp Ala Val His Glu Phe Thr Asp Trp
    2825                2830                2835

Leu His Ser Ala Lys Glu Glu Leu His Arg Trp Ser Asp Met Ser
    2840                2845                2850

Gly Asp Ser Ser Ala Thr Gln Lys Lys Leu Ser Lys Ile Lys Glu
    2855                2860                2865

Leu Ile Asp Ser Arg Glu Ile Gly Ala Ser Arg Leu Ser Arg Val
    2870                2875                2880

Glu Ser Leu Ala Pro Glu Val Lys Gln Asn Thr Thr Ala Ser Gly
    2885                2890                2895

Cys Glu Leu Met His Thr Glu Met Gln Ala Leu Arg Ala Asp Trp
    2900                2905                2910

Lys Gln Trp Glu Asp Ser Val Phe Gln Thr Gln Ser Cys Leu Glu
    2915                2920                2925

Asn Leu Val Ser Gln Met Ala Leu Ser Glu Gln Glu Phe Ser Gly
    2930                2935                2940

Gln Val Ala Gln Leu Glu Gln Ala Leu Glu Gln Phe Ser Ala Leu
    2945                2950                2955

Leu Lys Thr Trp Ala Gln Gln Leu Thr Leu Leu Glu Gly Lys Asn
    2960                2965                2970

Thr Asp Glu Glu Ile Val Glu Cys Trp His Lys Gly Gln Glu Ile
    2975                2980                2985

Leu Asp Ala Leu Gln Lys Ala Glu Pro Arg Thr Glu Asp Leu Lys
    2990                2995                3000

Ser Gln Leu Asn Glu Leu Cys Arg Phe Ser Arg Asp Leu Ser Thr
    3005                3010                3015

Tyr Ser Gly Lys Val Ser Gly Leu Ile Lys Glu Tyr Asn Trp
    3020                3025                3030

## Description

The present invention relates to gene expression in normal cells and cells of tumors and particularly to a novel marker protein associated with epithelial tumors, e.g., ovarian, mammary, stomach, kidney, thyroid, and lung carcinomas, Drop1, a dystrophin-related protein as well as a nucleic acid molecule encoding Drop1. Furthermore, the present invention relates to various diagnostic and therapeutic uses based on the finding that the expression of Drop1 is downregulated in epithelial tumors.

Tumorigenesis represents a complex multistage process in which genetic changes and environmental factors are thought to deregulate the cellular processes that control cell proliferation and differentiation. Among the gynaecological tumors, ovarian carcinoma has the highest mortality. It typically metastasizes to the peritoneal cavity. A comprehensive biological characterisation is crucial to improve the prognosis for cancer, e.g., ovarian cancer patients. Though morphological characterisation of the tumor in many cases gives a good hint towards prognosis and prediction of the response to specific therapies, its accuracy is limited and fails in many cases. Especially some tumors of low malignant potential turn out to be much more dangerous than initially thought. Therefore, markers are important which define the malignancy by its biological characteristics on the level of gene expression. These markers can also predict the possible success of a chemotherapy. Im mammary carcinoma, for instance, tumors overexpressing HER2/neu respond better to chemotherapy involving a taxane and anthracyclin than to one using alkylating agents. Nuclear proteins interacting with chemotherapeutic agents might also be involved in the cytotoxic mechanisms of these drugs, or their modified expression might be responsible for the failure of the treatment. In summary, the use of reliable diagnostic molecular markers would be helpful for an understanding of the molecular basis of tumors, e.g. ovarian and mammary carcinomas, for distinguishing benign from malign tissue and for grading and staging carcinoma. It can be expected that such markers are also useful for the development of novel therapeutic avenues for cancer treatment.

Thus, the technical problem underlying the present invention is to provide means for diagnosis and therapy of tumors, in particular epithelial carcinomas, which overcome the disadvantages of the prior art diagnostic and therapeutic methods.

The solution to said technical problem is achieved by providing the embodiments characterized in the claims.

During the experiments leading to the present invention a gene encoding a novel protein, Drop1, was found, which is downregulated in most cases of several types of carcinoma. Its expression is downregulated 14- to 210-fold in 85% of the ovarian and mammary carcinomas investigated. Similar downregulation was observed in a cancer profiling array for uterine, stomach, kidney, lung, and thyroid carcinomas. The Drop1 mRNA is present in only two out of eight ovarian carcinoma cell lines. The Drop1 gene comprises 56 exons in a genomic sequence of 180 kb. Starting from a differential display fragment of 680 bp, the 10.5 kb mRNA sequence was completed using RACE and an ovarian cDNA library as a template. The results were confirmed by overlapping PCR products using total cDNA from ovarian epithelial cells. Northern blots also show a diffuse band at 10 - 11 kb, mainly expressed in testes and in ovary. This was confirmed by in situ hybridisation. The gene is located in the chromosome region 6q25, where a loss of heterozygosity has been described for several types of carcinomas. Therefore, deletions may also cause down-regulation of this gene. The ORF predicts a protein of 349 kDa with a pI of 5.7, the sequence of which is only distantly related to muscle dystrophin, yet the overall structure is similar. It features two calponin homology domains and 12 spectrin-like repeats. Such domains are also found in muscle dystrophin. Their loss or truncation results in progressive muscular dystrophy with frequently fatal consequences. Drop1, in addition, displays an AAA domain of some 220 amino acid residues, which characterizes a large family of ATPases acting as ATP-dependent protein clamps. Dystrophin is present at the cytoplasmic membrane, where it integrates proteins into sterically fixed complexes. Drop1, in contrast, is located at or in the nucleus, where it may have a similar integrating function for nuclear protein complexes. Drop1 malfunction may impede cell cycle regulation, centrosome orientation and chromosome distribution. Dysfunction of mechanisms regulating these processes occur during pathogenesis and are key problems in malignant disease.

During the experiments resulting in the present invention it has been shown that Drop1 has high homology in an overlap to recently published proteins with unknown functions called enaptin (EMBL/Genbank/DDBJ databases AF535142), Nesprin-1 (Zhang et al., Genomics 80(5),pp. 473-481 (2002)), KIAA1756 (EMBL/Genbank/DDBJ databases AB051543) and Syne-1 (EMBL/Genbank/DDBJ databases AY135172). Furthermore, some sequences having homologies to Drop1 are shown in WO-A-02/40715. However, this international publication comprises a huge number of sequences without any specific relation to a disease. It is not plausible that all these sequences play the same or any role in such a large number of mentioned diseases.

In summary, Drop1 is a novel gene downregulated in epithelial tumors with a broad range of characteristics relevant for tumor biology. Downregulation, e.g. by promotor methylation, deletion, or truncation is expected to contribute to the development of carcinomas. Thus, Drop1 is very promising as a prognostic as well as a predictive marker.

### Brief description of the drawings

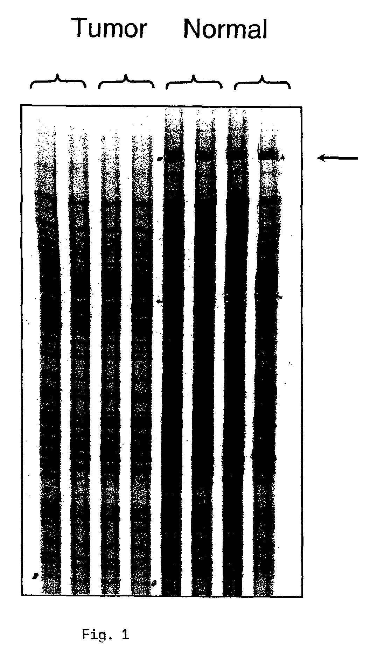

### Figure 1: Differential display with cDNA from normal and tumor tissue of patient 31, using anchor primer G and arbitrary primer DOC, generated a cDNA fragment 31N/GDOC/1 dd (arrow)

To avoid artefacts, RNA from both sources was twice reversely transcribed, and two differential display experiments were performed with each of the resulting four cDNAs, yielding eight samples run in parallel on the same gel for comparison. The figure shows the autoradiogram of the bands. The differentially expressed band was excised from the gel, eluted, amplified and cloned into pCRII-TOPO.

### Figure 2: Mutitissue Northern Blot hybridized with a randomly promed probe using the insert of clone 3R9 (see Fig. 6) as a template

### Figure 3: Autoradiogram of a cancer profiling array (BD Biosciences Clontech, Cat.# 7841-1 Lot #2040887) including amplified and normalized cDNA from 241 tumor (C) and corresponding normal tissues (N) from individual patients, spotted side by side

Boxed areas contain three cDNA spots from the same patient: top left normal, top right metastatic tissue, bottom right primary tumor. The array had previously been tested for equal loading of cDNA in the spots by hybridisation with a digoxigenin-labeled cDNA probe. Clone 3R9 was used to generate the radioactive Drop1 probe for hybridisation.

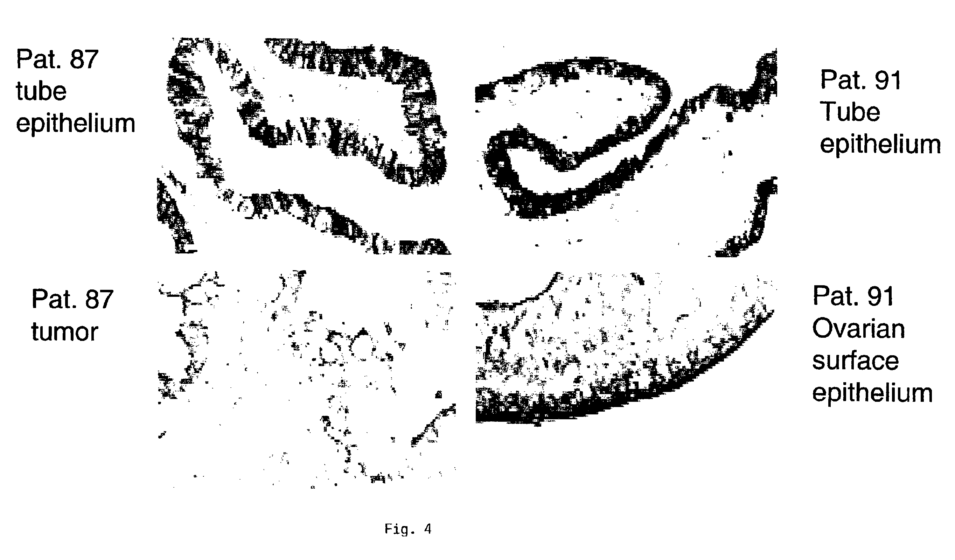

### Figure 4: Comparative in situ hybridisation of tissue sections with an antisense probe generated from clone 3R9

Right: both, tube epithelium (top) and ovarian surface epithelium (bottom) of patient 91 express Drop1 at a comparable level. Left: Tube epithelium (top) of patient 87 expresses Drop1 at a considerably higher level that the ovarian carcinoma (bottom) of the same patient.

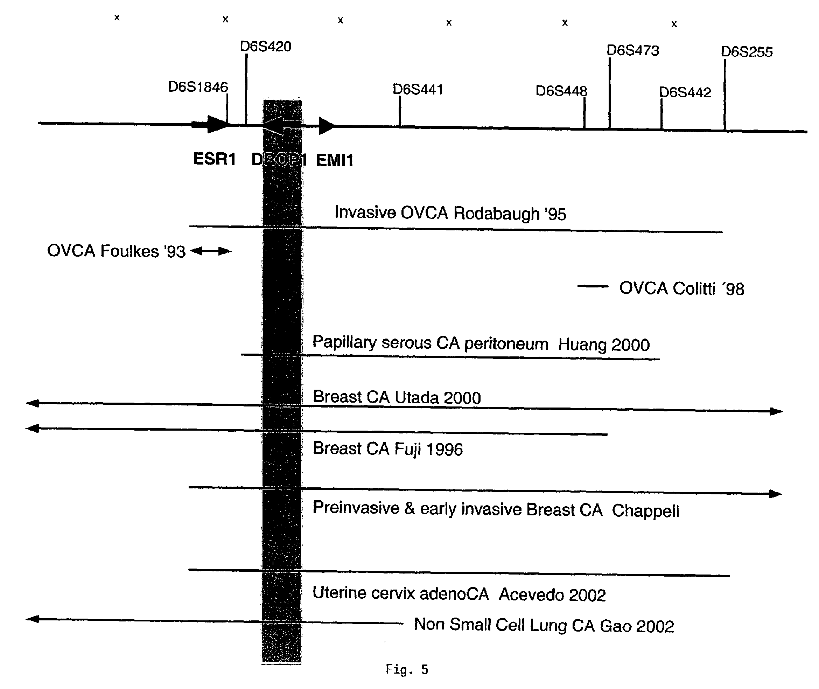

### Figure 5: Chromosome region 6q25.1, containing the Drop1 gene

Several groups have reported loss of heterozygosity for this region in ovarian, breast, uterine, and non small cell lung cancer, as indicated. Top line: chromosomal markers. Estrogen receptor 1 (ESR1, alpha) and early mitotic inhibitor 1(EMI1) are expressed from the plus strand (>), Drop1 from the minus strand (<).

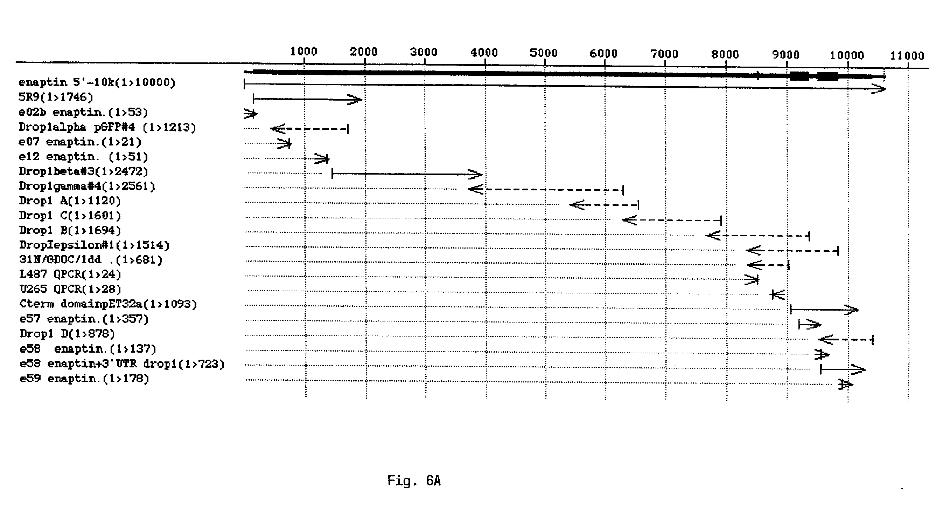

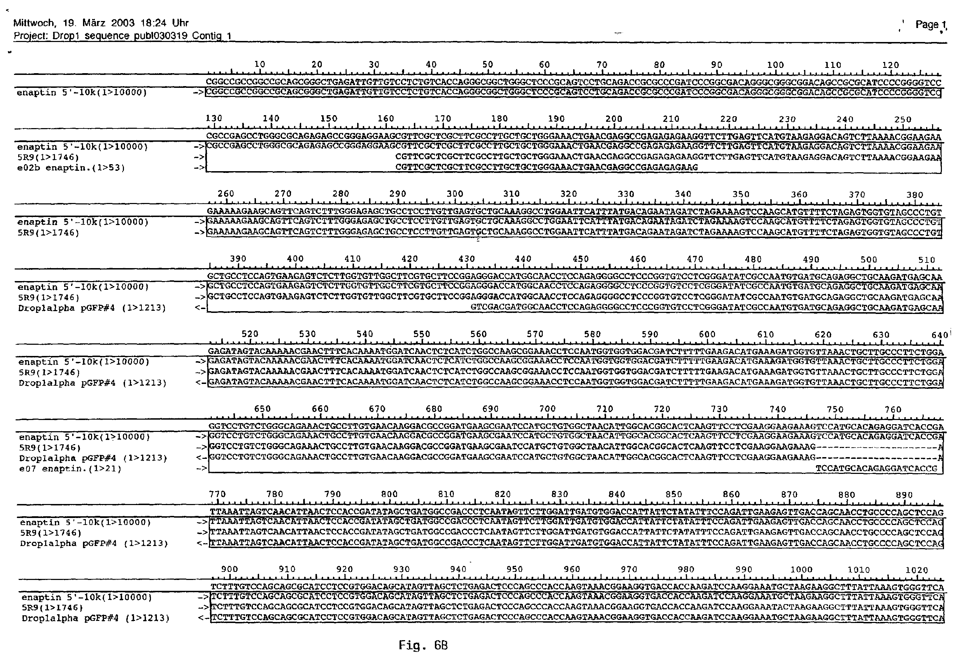

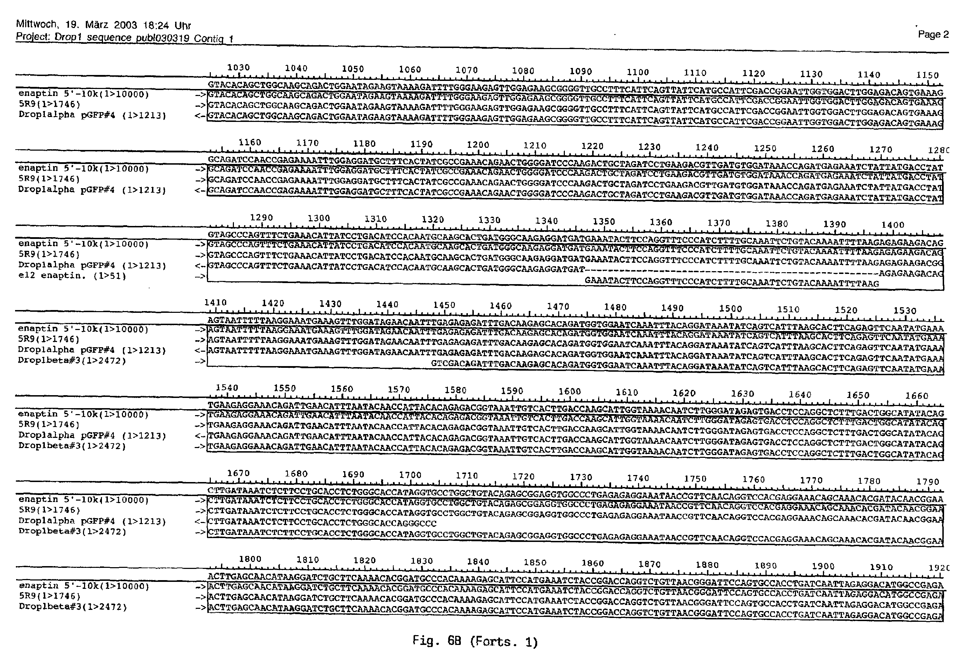

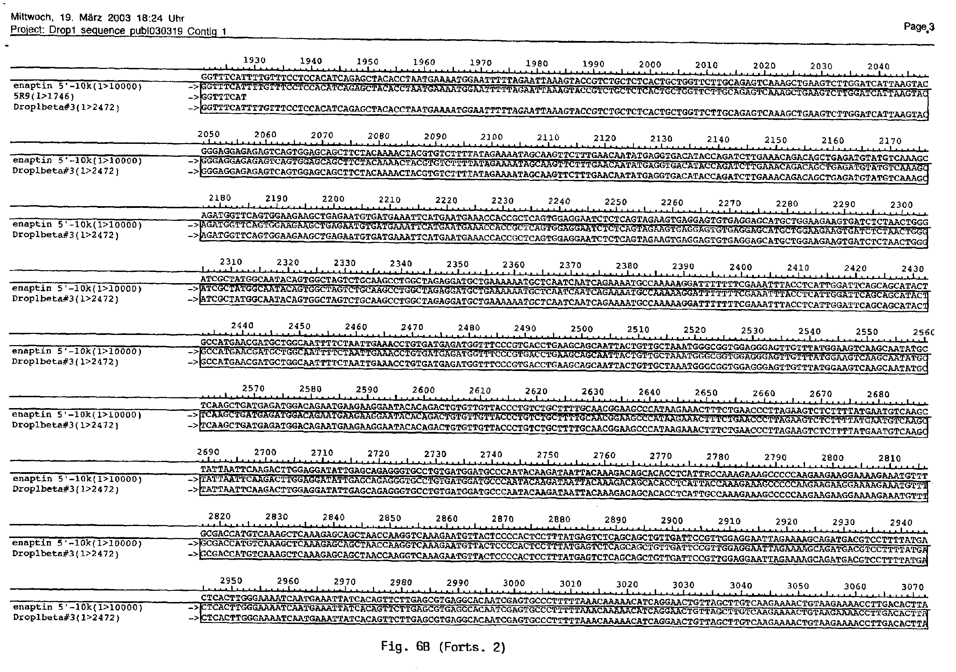

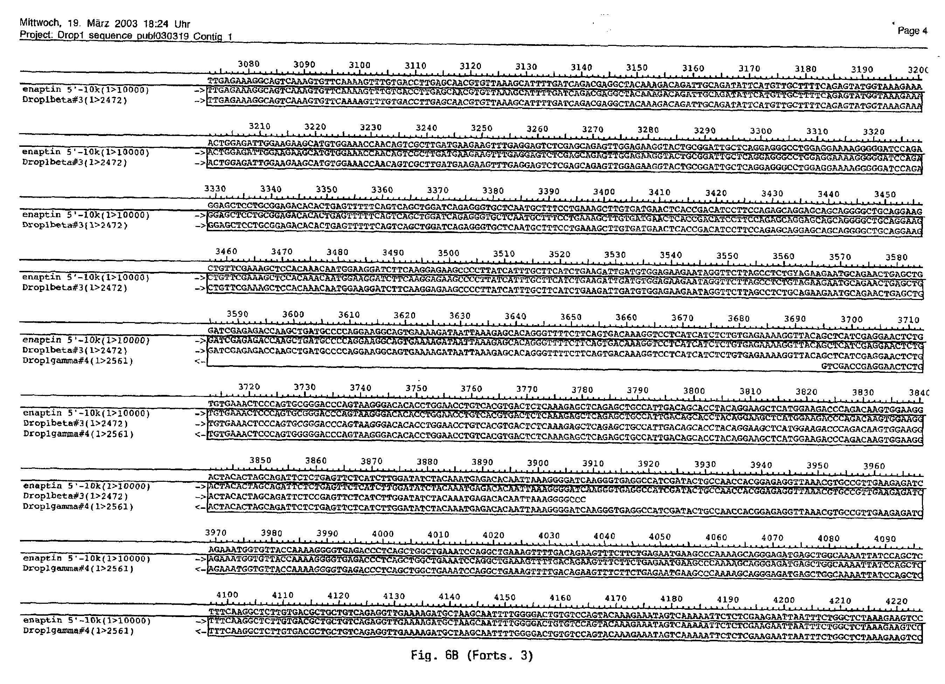

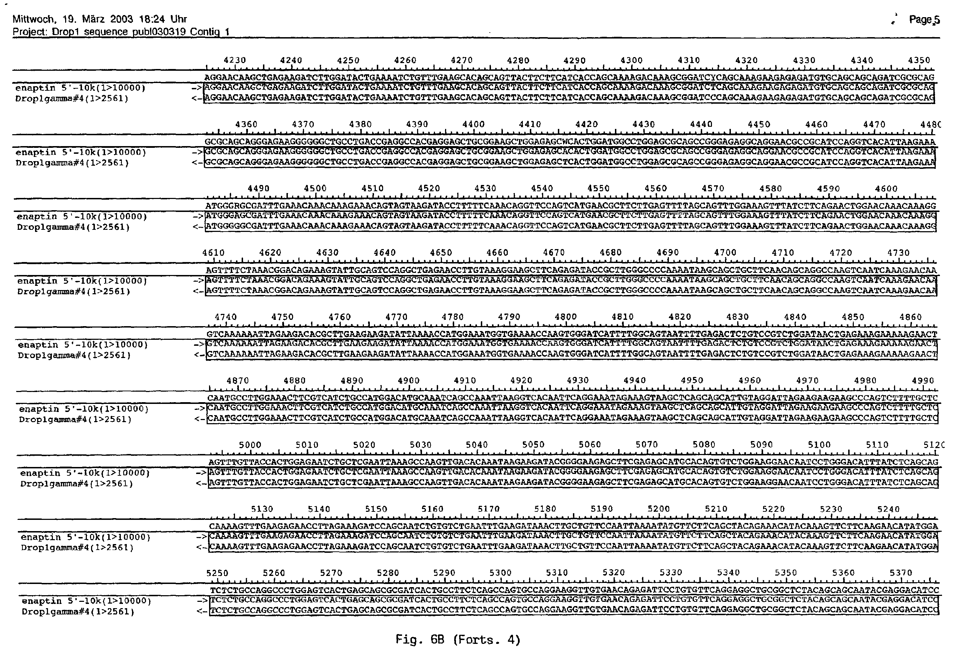

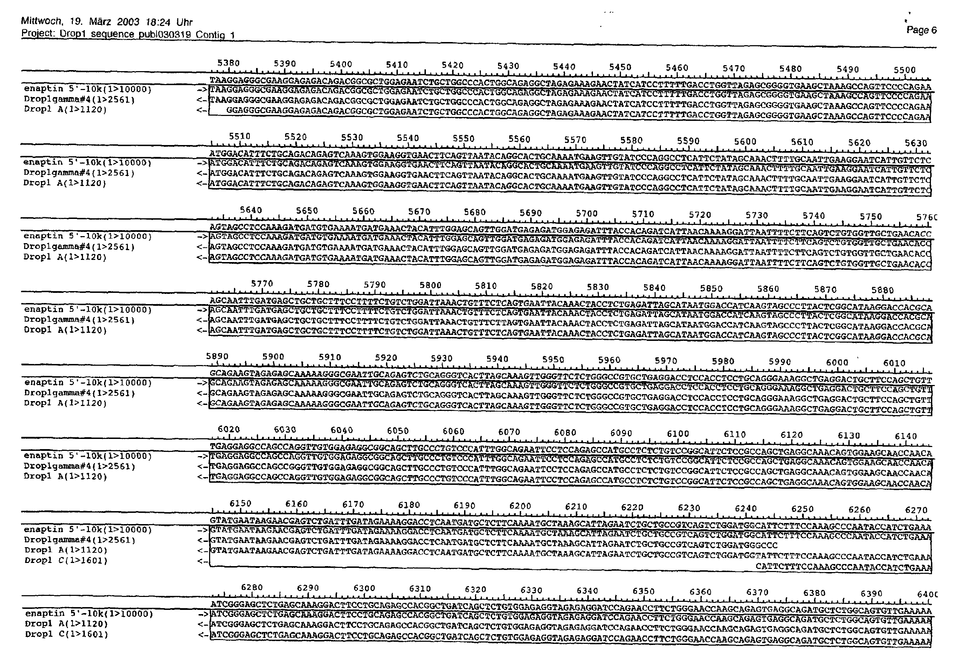

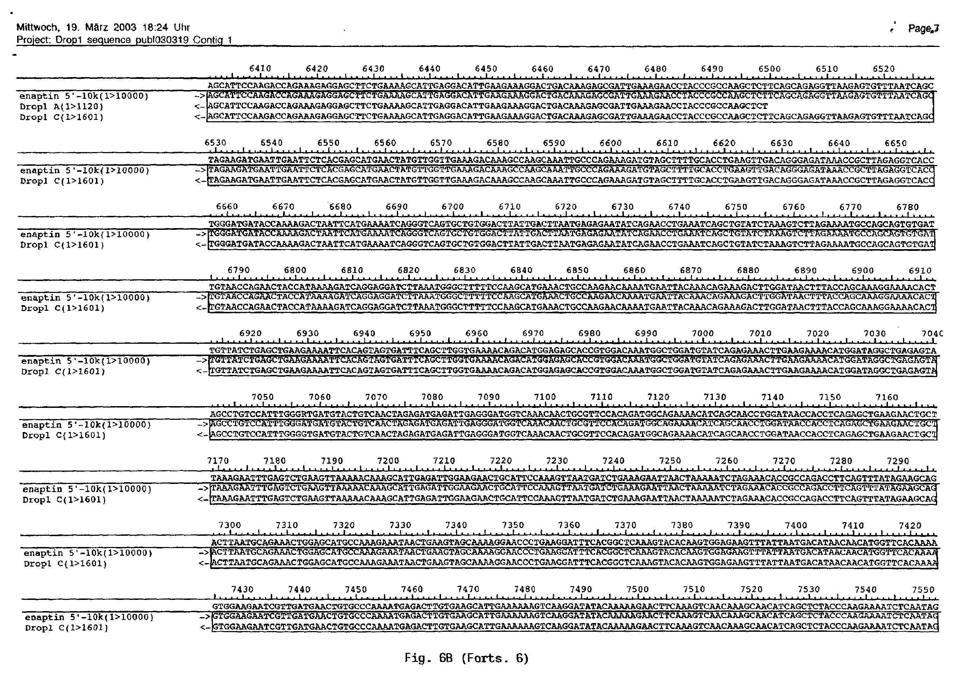

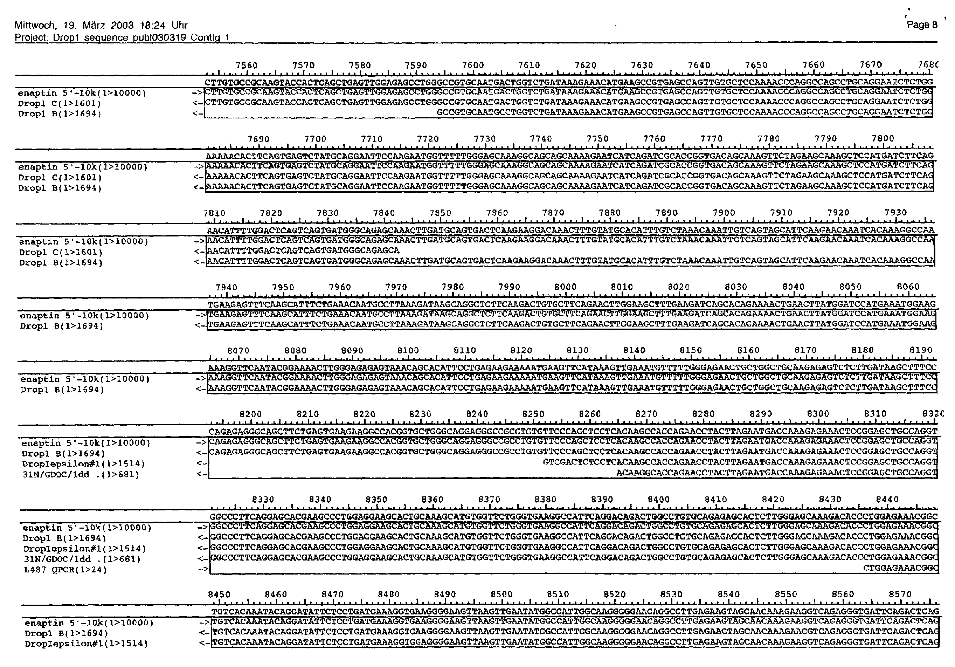

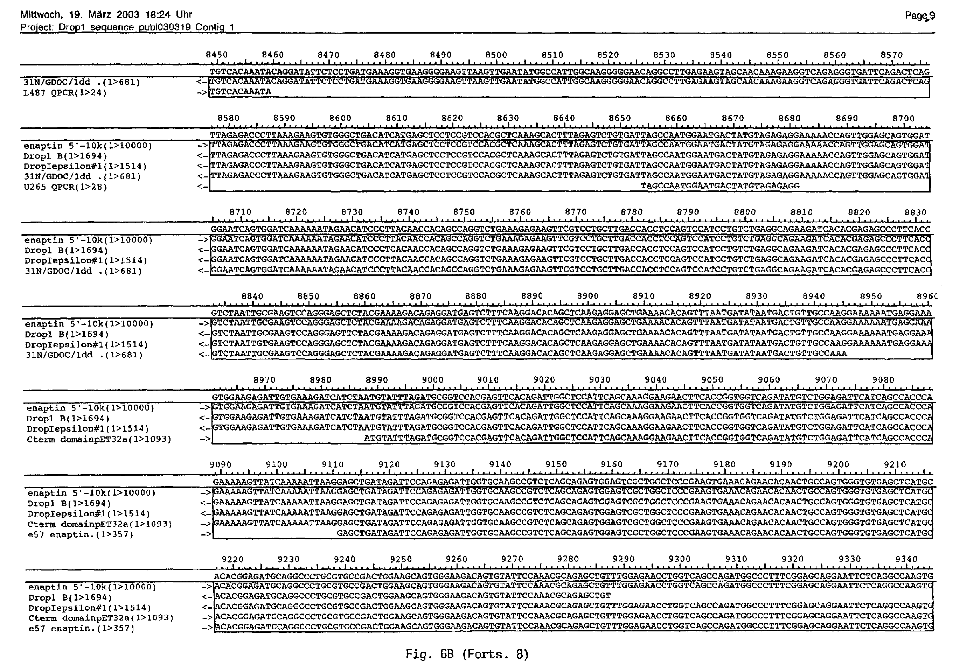

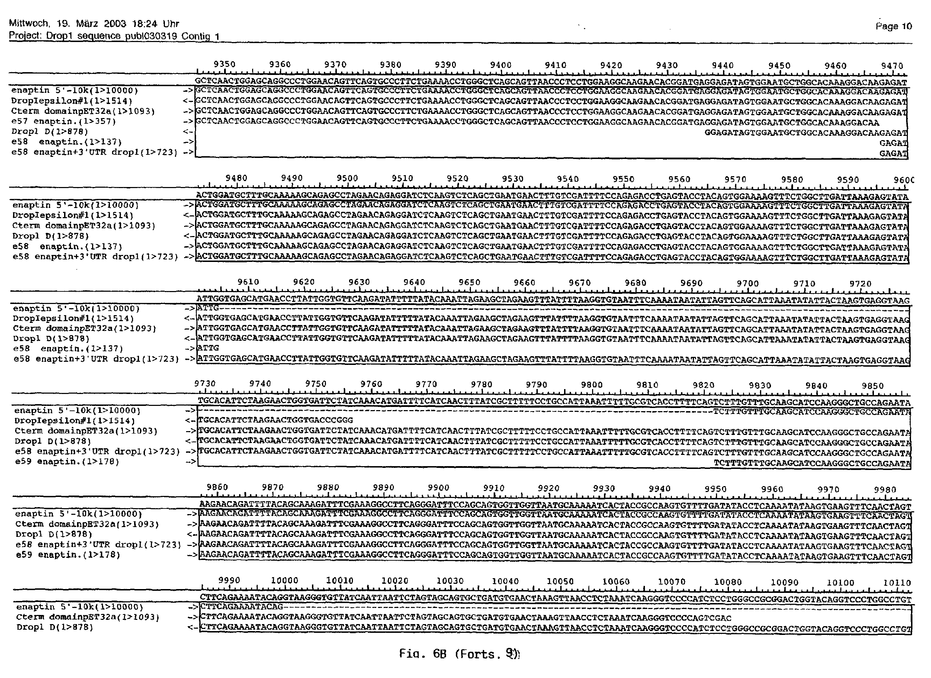

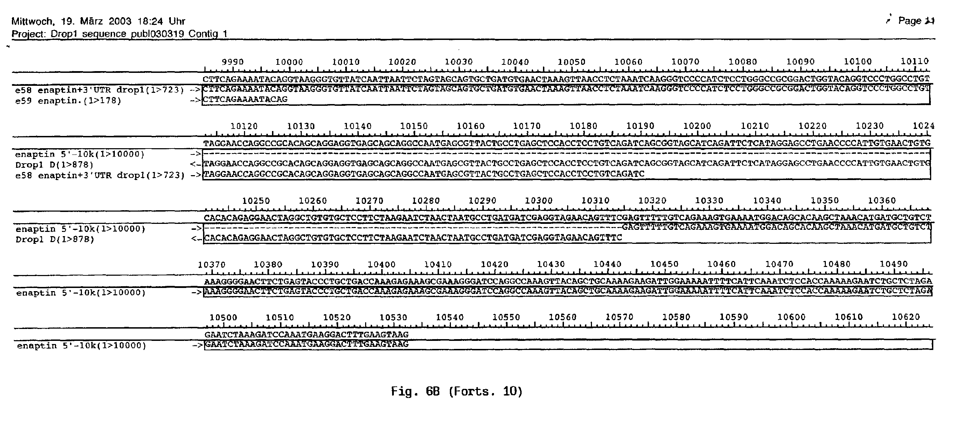

### Figure 6: Sequence details for Drop1

The figure shows the location in the contig of sequences mentioned in the text (A). The sequence was established starting with the 681 bp of the differential display fragment 31N/GDOC/1 dd. Primers L467 and U285 (their direction inverted in the final ORF) were derived from it and used throughout for quantitative PCR. "RACE walking" experiments were performed, based on a commercial RACE ovarian library. Of these, 5R9 marks the 5' region of drop1, 3R9 was used to generate the probes for in situ hybridisation, for multitissue Northern blots, and for the cancer profiling array. For validation of these sequences by overlapping PCR, total cDNA from SKOV-3 ovarian carcinoma cells was used as a template (Drop 1A,C,B,D). The same template yielded the clones used in bacterial and mammalian vectors for expression of domains (Drop1 alpha, beta, gamma, epsilon, and C-terminal domain). In total, more than 70 clones were aligned. Only upon completion of the Drop1 structure the enaptin sequence became available, of which here the 5'10 000 bp are shown (B). For ease of comparison the exons are designated accordingly. Drop1 is encoded by the 5'region (exons 2 to 58) of the enaptin gene (147 exons). The Drop1 mRNA and protein, however, are entities different from enaptin. Enaptin exons 07 and 12 are not used in Drop1, and enaptin-exon 58 is read differently: for Drop1 it is read beyond the enaptin-exon58-intron transition. It carries the stop codon for the ORF and a long 3' UTR that includes the unused sequence of enaptin-exon 59.

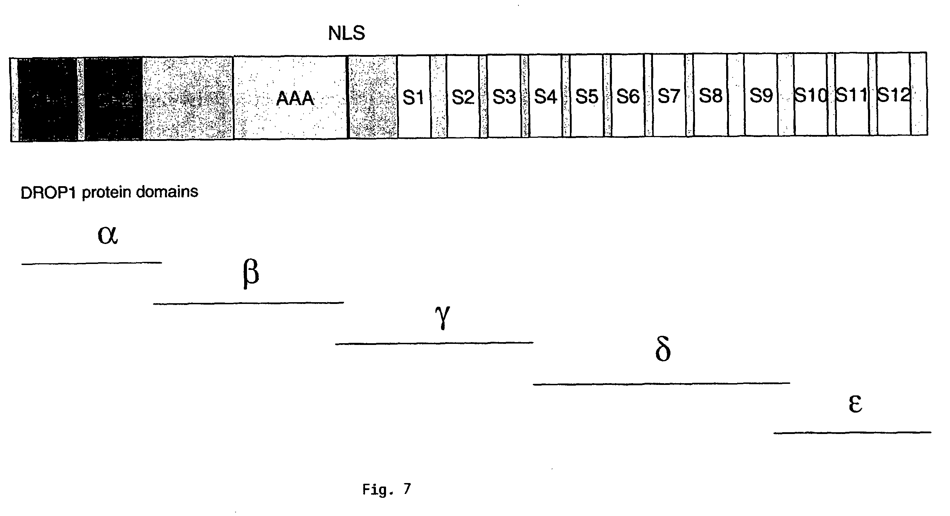

### Figure 7: Drop1 protein domains and overlapping expression clones

CH1, 2: calponin homology domains, AAA: ATPase domain, NLS: putative nuclear localization signal, S1 to S12: spectrin-like domains. alpha to epsilon: overlapping clones for expression from mammalian vectors.

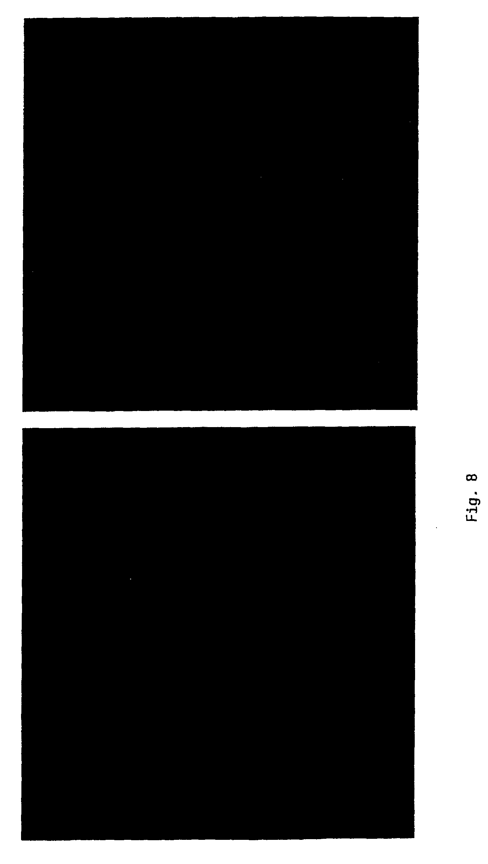

### Figure 8: Nuclear fluorescence of COS-7 cells transformed with GFP-Drop1a including the two calponin homology domains at the aminoterminus of Drop1

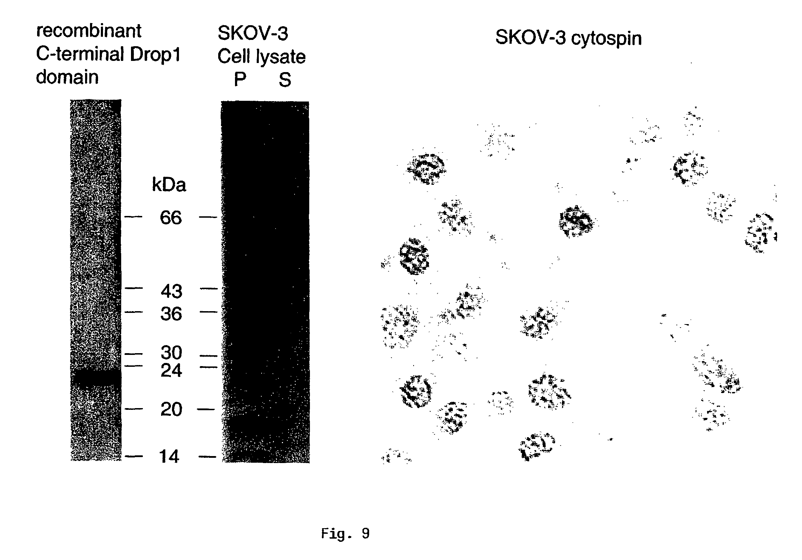

### Figure 9: Monoclonal antibody mAB2.9, raised to the recombinant carboxyterminal spectrin-like domain of Drop1, recognizes this recombinant domain on immunoblots (left)

On blots of SKOV-3 ovarian carcinoma cell lysates (center) it stains a high molecular band and various fragments in the pellet (P), and a high molecular weight band and various fragments in the pellet (P), and a large protein in the supernatant (S). In cytospins of the same cells it stains nuclei (right).

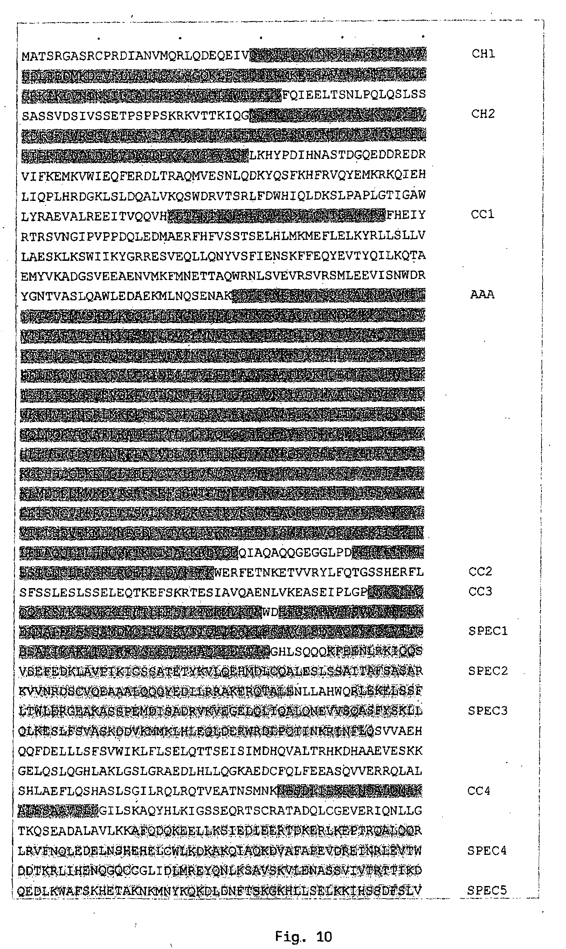

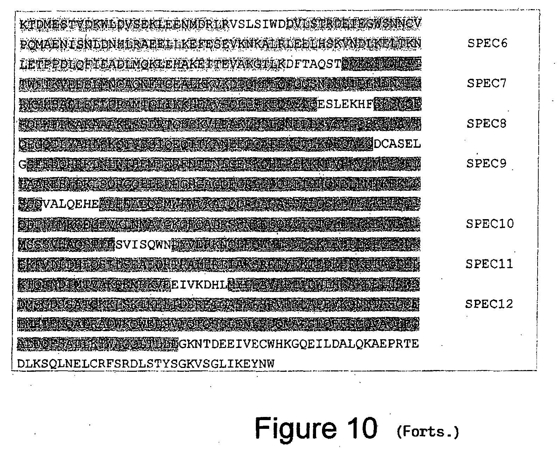

### Figure 10: Predicted amino acid sequence of Drop1 and predicted protein domains

The ORF encodes a protein of 3032 amino acid residues. Analysis using SMART (Simple Modular Architecture Research Tool) software indicates two calponin homology (CH) domains, an AAA, four coiled coil (CC) motifs and twelve spectrin-like (SPEC) regions, as highlighted in gray in the sequence and indicated in the margin.

The present invention relates to a nucleic acid molecule encoding the human dystrophin-related polypeptide Drop1 or a polypeptide exhibiting a biological property of Drop1, which is:
(a) a nucleic acid molecule encoding a Drop1 polypeptide that consists of the amino acid sequence as depicted in Figure 10;
(b) a nucleic acid molecule consisting of the nucleotide sequence as depicted in Figure 6B;
(c) a nucleic acid molecule the sequence of which differs from the sequence of a nucleic acid molecule of (a) or (b) due to the degeneracy of the genetic code;
(d) a nucleic acid molecule, which represents a fragment of a nucleic acid molecule specified in (a) to (c); or
(e) a nucleic acid molecule complementary to the nucleic acid molecule specified in (a) to (d).

As used herein, a polypeptide exhibiting a biological property of Drop1 is understood to be a polypeptide having at least one of the activities of Drop1 as illustrated in the Examples, below, i.e. at least one biological activity of an ATPase.

In a first embodiment, the invention provides a nucleic acid molecule encoding a Drop1 polypeptide that consists of the amino acid sequence as depicted in Figure 10.

The nucleic acid molecules of the invention can be both DNA and RNA molecules. Suitable DNA molecules are, for example, genomic or cDNA molecules. It is understood that all nucleic acid molecules encoding all or a portion of Drop1 are also included, as long as they encode a polypeptide with biological activity. The nucleic acid molecules of the invention can be isolated from natural sources or can be synthesised according to known methods.

The nucleic acid molecules of the present invention also include molecules which differ from the nucleic acid molecules with sequences shown in Figure 6B due to the degeneracy of the genetic code.

In a further embodiment, the present invention provides nucleic acid molecules which comprise fragments of the nucleic acid molecules described above encoding a polypeptide of the invention. "Fragments" are understood to be parts of the nucleic acid molecules that are long enough to encode one of the described polypeptids. These fragments also comprise nucleic acid molecules specifically hybridizing to transcripts of the nucleic acid molecules of the invention. These nucleic acid molecules can be used, for example, as probes or primers in the diagnostic assay and/or kit described below and, preferably, are oligonucleotides having a length of at least 10, in particular of at least 15 and particularly preferred of at least 25 contiguous nucleotides of the nucleic acid molecule of the invention. The nucleic acid molecules and oligonucleotides (fragments) of the invention can also be used, for example, as primers for a PCR reaction.

In a still further embodiment, the present invention provides nucleic acid molecules which are complementary to the nucleic acid molecules of the invention.

Generally, by means of conventional molecular biological processes it is possible (see, e.g., Sambrook et al., supra) to introduce different mutations into the nucleic acid molecules of the invention. As a result Drop1 polypeptides or Drop1 related polypeptids with possibly modified biological properties are synthesised. One possibility is the production of deletion mutants in which nucleic acid molecules are produced by continuous deletions from the 5'- or 3'-terminal of the coding DNA sequence and that lead to the synthesis of polypeptids that are shortened accordingly. Another possibility is the introduction of single-point mutation at positions where a modification of the amino aid sequence influences, e.g., the proteolytic properties. By this method muteins can be produced, for example, that possess a modified Kₘ-value or that are no longer subject to the regulation mechanisms that normally exist in the cell, e.g. with regard to allosteric regulation or covalent modification.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The polypeptids encoded by the various variants of the nucleic acid molecules of the invention show certain common characteristics, such as ATPase activity, molecular weight, immunological reactivity or conformation or physical properties like the electrophoretical mobilty, chromatographic behavior, sedimentation coefficients, solubility, spectroscopic properties, stability, pH optimum, temperature optimum.

The invention furthermore relates to recombinant vectors containing the nucleic acid molecules of the invention. Preferably, they are plasmids, cosmids, viruses, bacteriophages and other vectors usually used in the field of genetic engineering. Vectors suitable for use in the present invention include, but are not limited to the T7-based expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an mRNA in prokryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors of the invention. A host cell is understood to be an organism that is capable to take up *in vitro* recombinant DNA and, if the case may be, to synthesize the polypeptids encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to a polypeptide exhibiting a biological property of the human dystrophin-related polypeptide Drop1 and being encoded by the nucleic acid molecules of the invention, as well as to methods for their production, whereby, e.g., a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., an anti-Drop1 antibody, or, e.g., can be substantially purified by the one-step method described in Smith and Johnson, Gene 67; 31-40 (1988). These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art. These polypeptides are preferably in a substantially purified form.

The present invention also relates to an antibody that binds specifically to a Drop1 polypeptide or a related polypeptide as defined above. Cells producing said antibody have been deposited on March 26, 2003 according to the Budapest Treaty with the Deutsche Sammlung von Mikrorganismen und Zellkulturen, Mascheroder Weg, Braunschweig, Germany. The term "antibody", preferably, relates to antibodies which consist essentially of pooled monoclonal antibodies with different epitopic specifities, as well as distinct monoclonal antibody preparations. Monoclonal antibodies are made from an antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

For certain purposes, e.g. diagnostic methods, the antibody of the present invention can be detectably labeled, for example, with a radioisotope, a bioluminescent compound, a chemiluminescent compound, a fluorescent compound, a metal chelate, or an enzyme.

The invention also relates to a transgenic non-human animal such as transgenic mouse, rats, hamsters, dogs, monkeys, rabbits, pigs, C. elegans and fish such as torpedo fish comprising a nucleic acid molecule or vector of the invention, preferably wherein said nucleic acid molecule or vector is stably integrated into the genome of said non-human animal, preferably such that the presence of said nucleic acid molecule or vector leads to the expression of a Drop1 polypeptide (or related polypeptide) of the invention. Said animal may have one or several copies of the same or different nucleic acid molecules encoding one or several forms of a Drop1 polypeptide or mutant forms thereof. This animal has numerous utilities, including as a research model for development/progression of carcinomas and therefore, presents a novel and valuable animal in the development of therapies, treatment, etc. for carcinomas associated with downregulation of Drop1, e.g., epithelial tumors. Accordingly, in this instance, the non-human mammal is preferably a laboratory animal such as a mouse or rat.

Preferably, the transgenic non-human animal of the invention further comprises at least one inactivated wild type allele of the corresponding Drop1 encoding gene. This embodiment allows for example the study of the interaction of various mutant forms of Drop1 polypeptides on the onset of the clinical symtoms of a carcinoma. All the applications that have been herein before discussed with regard to a transgenic animal also apply to animals carrying two, three or more transgenes. It might be also desirable to inactivate Drop1 protein expression or function at a certain stage of development and/or life of the transgenic animal. This can be achieved by using, for example, tissue specific, developmental and/or cell regulated and/or inducible promoters which drive the expression of, e.g., an antisense or ribozyme directed against the RNA transcript encoding the Drop1 encoding mRNA; see also supra. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of a mutant Drop1 protein may be controlled by such regulatory elements.

Furthermore, the invention also relates to a transgenic mammalian cell which contains (preferably stably integrated into its genome) a nucleic acid molecule according to the invention or part thereof, wherein the transcription and/or expression of the nucleic acid molecule or part thereof leads to reduction of the synthesis of a Drop1 protein. In a preferred embodiment, the reduction is achieved by an anti-sense, sense, ribozyme, co-suppression and/or dominant mutant effect. "Antisense" and "antisense nucleotides" means DNA or RNA constructs which block the expression of the naturally occurring gene product.

The provision of the nucleic acid molecule according to the invention opens up the possibility to produce transgenic non-human animals with a reduced level of a Drop1 protein as described above. Techniques how to achieve this are well known to the person skilled in the art. These include, for example, the expression of antisense-RNA, ribozymes, of molecules which combine antisense and ribozyme functions and/or of molecules which provide for a co-suppression effect. When using the antisense approach for reduction of the amount of Drop1 proteins in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a Drop1 protein. In this case the homology is preferably higher than 80%, particularly higher than 90% and still more preferably higher than 95%. The reduction of the synthesis of a polypeptide according to the invention in the transgenic mammalian cells can lead to various physiological, developmental and/or morphological changes.

Thus, the present invention also relates to transgenic non-human animals comprising the above-described transgenic cells. These may show, for example, a deficiency in Drop1 due to the stable or transient presence of a foreign DNA resulting in at least one of the following features:
(a) disruption of (an) endogenous gene(s) encoding Drop1;
(b) expression of at least one antisense RNA and/or ribozyme against a transcript comprising a nucleic acid molecule of the invention;
(c) expression of a sense and/or non-translatable mRNA of the nucleic acid molecule of the invention;
(d) expression of an antibody of the invention;
(e) incorporation of a functional or non-functional copy of the regulatory sequence of the invention; or
(f) incorporation of a recombinant DNA molecule or vector of the invention.

Methods for the production of a transgenic non-human animal of the present invention, preferably transgenic mouse, are well known to the person skilled in the art. Such methods, e.g., comprise the introduction of a nucleic acid molecule or vector of the invention into a germ cell, an embryonic cell, stem cell or an egg or a cell derived therefrom. The non-human animal can be used in accordance with a screening method of the invention described herein and may be a non-transgenic healthy animal, or may have a disorder, preferably a disorder caused by at least one mutation in the Drop1 protein. Such transgenic animals are well suited for, e.g., pharmacological studies of drugs in connection with mutant forms of the above described Drop1 polypeptide. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe; see supra.

In a further aspect, the invention relates to a method of identifying activators/agonists of a Drop1 polypeptide comprising the steps of: (a) incubating a candidate compound with a polypeptide of the invention, b) assaying a biological activity, and (c) determining if a biological activity of the polypeptide of the invention has been altered.

Preferably, the screening for these compounds involves producing appropriate cells which express Drop1, either as a secreted protein or on the cell membrane. Preferred cells include cells from mammals, yeast, Drosophila, or E. coli. Cells expressing Drop1 (or cell membrane containing the expressed polypeptide) are then preferably contacted with a test compound potentially containing the molecule to observe binding, stimulation, or inhibition of activity of Drop1.

The assay may simply test binding of a candidate compound to Drop1, wherein binding is detected by a label, or in an assay involving competition with a labeled competitor. Further, the assay may test whether the candidate compound results in a signal generated by binding to Drop1.

Alternatively, the assay can be carried out using cell-free preparations, polypeptide/molecule affixed to a solid support, chemical libraries, or natural product mixtures. The assay may also simply comprise the steps of mixing a candidate compound with a solution containing Drop1, measuring Drop1/molecule activity or binding, and comparing the Drop1/molecule activity or binding to a standard.

Preferably, an ELISA assay can measure Drop1 level or activity in a sample (e.g., biological sample) using a monoclonal or polyclonal antibody. The antibody can measure Drop1 level or activity by either binding, directly or indirectly, to Drop1 or by competing with Drop1 for a substrate. All of these above assays can be used as diagnostic or prognostic markers. The molecules discovered using these assays can be used to treat disease or to bring about a particular result in a patient (e.g., elimination of an epithelial tumor or stop of progression of tumor growth) by activating the Drop1 molecule. Moreover, the assays can discover agents which may enhance the production of Drop1 from suitably manipulated cells or tissues.

The candidate compound may be a single compound or a plurality of compounds. The term "plurality of compounds" in a method of the invention is to be understood as a plurality of substances which may or may not be identical.

Said compound or plurality of compounds may be chemically synthesized or microbiologically produced and/or comprised in, for example, samples, e.g., cell extracts from, e.g., plants, animals or microorganisms. Furthermore, said compound(s) may be known in the art but hitherto not known to be capable of activating Drop1 polypeptides. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994) and in the appended examples. The plurality of compounds may be, e.g., added to the reaction mixture, culture medium, injected into a cell or otherwise applied to the transgenic animal. The cell or tissue that may be employed in the method of the invention preferably is a host cell, mammalian cell or non-human transgenic animal of the invention described in the embodiments hereinbefore.

If a sample containing a compound or a plurality of compounds is identified in the method of the invention, then it is either possible to isolate the compound from the original sample identified as containing the compound capable of activating Drop1, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical.

Several methods are known to the person skilled in the art for producing and screening large libraries to identify compounds having specific affinity for a target. These methods include the phage-display method in which randomized peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labeled receptor and scanned for label to identify polymers binding to the receptor. The synthesis and screening of peptide libraries on continuous cellulose membrane supports that can be used for identifying binding ligands of the polypeptide of the invention and thus possible activators is described, for example, in Kramer, Methods Mol. Biol. 87 (1998), 25-39.

More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the polypeptides according to the invention or nucleic acid molecules encoding such molecules are, for example, the in vitro screening with the phage display system as well as filter binding assays or "real time" measuring of interaction using, for example, the BIAcore apparatus (Pharmacia).

All these methods can be used in accordance with the present invention to identify activators/agonists of the Drop1 polypeptide or related polypeptide of the invention.

The nucleic acid molecule of the invention can also serve as a target for activators. Activators may comprise, for example, proteins that bind to the mRNA of a gene encoding a Drop1 polypeptide of the invention, thereby stabilizing the native conformation of the mRNA and facilitating transcription and/or translation, e.g., in like manner as Tat protein acts on HIV-RNA. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical interest, and for identifying unknown RNA targets for use in treating a disease. These methods and compositions can be used in screening for identifying compounds useful to alter expression levels of proteins encoded by a nucleic acid molecule. Alternatively, for example, the conformational structure of the RNA fragment which mimics the binding site can be employed in rational drug design to modify known antibiotics to make them bind more avidly to the target. One such methodology is nuclear magnetic resonance (NMR), which is useful to identify drug and RNA conformational structures. Still other methods are, for example, the drug design methods as described in WO 95/35367 or US-A-5,322,933, where the crystal structure of the RNA fragment can be deduced and computer programs are utilized to design novel binding compounds which can act as antibiotics.

The identification of nucleic acid molecules which encode polypeptides which interact with the Drop1 polypeptides described above can also be achieved, for example, as described in Scofield (Science 274 (1996), 2063-2065) by use of the so-called yeast "two-hybrid system". In this system the polypeptide encoded by a nucleic acid molecule according to the invention or a smaller part thereof is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of a Drop1 polypeptide of the invention, the complex is able to direct expression of the reporter gene. In this way the nucleic acid molecules according to the invention and the encoded peptide can be used to identify peptides and proteins interacting with Drop1 protein.

Once the transacting factor is identified, modulation of its binding to or regulation of expression of the Drop1 polypeptide of the invention can be pursued, beginning with, for example, screening for inhibitors against the binding of the transacting factor to the protein of the present invention. Activation of Drop1 proteins could then be achieved in animals by applying the transacting factor or the gene encoding it, e.g. in an expression vector. Furthermore, upon identification of the transacting factor, further components in the pathway leading to activation (e.g. signal transduction) or repression of a gene involved in the control of Drop1 then can be identified. Modulation of the activities of these components can then be pursued, in order to develop additional drugs and methods for enhancing the activity/amount of Drop1. Thus, the present invention also relates to the use of the two-hybrid system as defined above for the identification of activators of Drop1.

The compounds isolated by the above methods also serve as lead compounds for the development of analog compounds. The analogs should have a stabilized electronic configuration and molecular conformation that allows key functional groups to be presented to the Drop1 or its possible receptor in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above.

Once the described compound has been identified and obtained, it is preferably provided in a therapeutically acceptable form.

Accordingly, the present invention also relates to a pharmaceutical composition comprising a nucleic acid molecule, polypeptide, recombinant vector, antibody, or activator/agonist according to the present invention and optionally a pharmaceutically acceptable excipient, diluent or carrier.

The nucleic acid molecule suitable for pharmaceutical or diagnostic purposes is defined to be
(a) a nucleic acid molecule encoding a Drop1 polypeptide that comprises the amino acid sequence as depicted in Figure 10;
(b) a nucleic acid molecule comprising the nucleotide sequence as depicted in Figure 6B;
(c) a nucleic acid molecule encoding a polypeptide the sequence of which shows at least 65% idendity to the amino acid sequence of the polypeptide encoded by a nucleic acid molecule specified in (a) or (b);
(d) a nucleic acid molecule the sequence of which differs from the sequence of a nucleic acid molecule of (a) to (c) due to the degeneracy of the genetic code;
(e) a nucleic acid molecule, which represents a fragment of a nucleic acid molecule specified in (a) to (d); or
(f) a nucleic acid molecule complementary to the nucleic acid molecule specified in (a) to (e).

The present invention also provides the pharmaceutical or diagnostic use of nucleic acid molecules encoding a polypeptide the amino acid sequence of which shows an identity of at least 80%, in particular an identity of at least 90%, preferably of at least 95%, 96%, 97%, 98% or 99% to the amino acid sequence of the polypeptide of Figure 10. The amino acid sequences of such polypeptides are characterized by deletion, substitution and/or insertion of amino acid residue(s) compared to the amino acid sequence shwon in Fig. 10 or the result of recombination. They can be naturally occurring variations, for example sequences from other organisms, or mutations that cam either occur naturally or that have been introduced by genetic mutagenesis. In order to identify and isolate such nucleic acid molecules, the molecules of the invention or parts thereof can be used, for example by means of hybridization. As a hybridization probe nucleic acid molecules can be used, for example, that have exactly or basically the nucleic acid sequence as depicted in Fig. 6B, or parts of these sequences. The fragments that have been used as hybridization probes can be synthetic fragments that were produced by means of conventional synthetic methods and the sequence of which basically corresponds to the sequence of the Fig. 6B sequence.

As used herein, the term "hybridisation" has the meaning of hybridisation under conventional hybridisation conditions, preferably under stringent conditions as described, for example, in Sambrook et al., Molecular Cloning, A Laboratory Manual 2^{nd} edition (1989) Cold Spring Harbor Laboratory Press, Cold Spring Harbor, NY. However, in certain cases, a hybridizing nucleic acid molecule can also be detected at lower stringency hybridisation conditions. Changes in the stringency of hybridisation and signal detection are primarily accomplished through the manipulation of formamide concentration (lower percentages of formamide result in lowered stringency), salt conditions, or temperature. For example, lower stringency conditions include an overnight incubation at 37°Cin a solution comprising 6X SSPE (20X SSPE = 3M NaCl; 9.2M NaH₂PO₄ 0.02M EDTA, pH7.4), 0.5% SDS, 30% formamide, 100 µg/ml salmon sperm blocking DNA, following by washes at 50°C with 1 X SSPE, 0.1% SDS. In addition, to achieve even lower stringency, washes performed following stringent hybridisation can be done at higher salt concentrations (e.g. 5X SSC). Variations in the above conditions may be accomplished through the inclusion and/or substitution of alternate blocking reagents used to suppress background in hybridisation experiments. The inclusion of specific blocking reagents may require modification of the hybridisation conditions described above, due to problems with compatibility.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the tumor, its localisation and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of an epithelial tumor, general health and other drugs being administered concurrently.

The delivery of the nucleic acid molecules of the invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above nucleic acid molecules include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tumor. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific epithelial tumors via specific cell-surface ligands.

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 97/00957.

In order to achieve expression only in the target organ, e.g., an epithelial tumor to be treated, the nucleic acid molecules of the present invention can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840).

The present invention also relates to the use of the above compounds of the invention for the preparation of a pharmaceutical composition for treatment of an epithelial tumor, preferably for the treatment of ovarian, mammary, stomach, kidney, thyroid, pancreas, cervical, testis or lung carcinomas.

Recombinant re-expression of a protein which got lost or lost its activity by mutations during tumor progression is a strategy which has been clinically evaluated for ovarian carcinoma. Wild-type P53 was re-introduced into mutant cells in a phase I clinical trial using a viral vector. This gave a proof of principle that gene therapy is a promising strategy in ovarian cancer. The predominantly intraperitoneal pattern of metastasising of ovarian carcinoma makes it prone for intraperitoneal application of the therapeutic agent which increases the safety of the therapy and should lead to only moderate toxicity of such therapies. Considering the size of Drop1, even transfection of defective cells with cDNA fragments may have a therapeutic effect. This strategy has already been used successfully with muscle dystrophies in animal models (Chamberlain, Hum. Mol. Genet. 11 (2002), 2355-2362; Scott et al., Neuromuscular Disord. 12 (2002), S23-S29). In addition, it can be expected that expression of Drop1 can be upregulated in tumors by therapeutic use of demethylating drugs.

Finally, the present invention also relates to a method of diagnosing an epithelial tumor or a susceptibility to an epithelial tumor in a subject comprising:
(a)determining the presence or amount of expression of the Drop1 polypeptide in a biological sample; and
(b) diagnosing an epithelial tumor or a susceptibility to an epithelial tumor based on the presence or amount of expression of the Drop1 polypeptide.

The present invention also relates to a diagnostic kit useful for the detection and/or characterization of an epithelial tumor or a predisposition to such a tumor, e.g., an ovarian or mammary carcinoma, containing a nucleic acid molecule, polypeptide and/or antibody of the invention. Such kits are useful for the detection of a target cellular component, which is Drop1 or, alternatively, Drop1 encoding mRNA, wherein an decreased concentration of Drop1 (compared to the concentration in normal tissue) or, alternatively, Drop1 encoding mRNA is indicative for a epithelial tumor or a disposition to such a tumor.

The Drop1 polypeptide or the corresponding mRNA, e.g. in biological fluids or tissues, may be detected directly in situ, e.g. by in situ hybridisation (e.g., according to the examples, below) or it may be isolated from other cell components by common methods known to those skilled in the art before contacting with a probe. Detection methods include Northern Blot analysis, RNase protection, in situ methods, e.g. in situ hybridisation, in vitro amplification methods (PCR, LCR, QRNA replicase or RNA-transcription/amplification (TAS, 3SR), reverse dot blot disclosed in EP-B1 O 237 362)), immunoassays, Western Blot and other detection assays that are known to those skilled in the art. Prferably, these detection methods are in an array formate.

The probe (e.g. a specific antibody or specific oligonucleotide) of the diagnostic kit can be detectably labeled. In a preferred embodiment, said kit contains an anti-Drop1 antibody and allows said diagnosis, e.g., by ELISA and contains the antibody bound to a solid support, for example, a polystyrene microtiter dish or nitrocellulose paper, using techniques known in the art. Alternatively, said kits are based on a RIA and contain said antibody marked with a radioactive isotope. In a preferred embodiment of the kit of the invention the antibody is labeled. Suitable antibody assay labels are known in the art and include enzyme labels, such as, glucose oxidase, and radioisotopes, such as iodine (¹²⁵I, ¹²¹I), carbon (¹⁴C), sulfur (³⁵S) _{,} tritium (³H), indium (¹¹²In), and technetium rhodamine, and biotin. In addition to assaying Drop1 levels in a biological sample, the polypeptide can also be detected in vivo by imaging. Antibody labels or markers for in vivo imaging of protein include those detectable by X-radiography, NMR or ESR. For X-radiography, suitable labels include radioisotopes such as barium or caesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. A protein-specific antibody or antibody fragment which has been labeled with an appropriate detectable imaging moiety, such as a radioisotope (for example, ¹³¹I, ¹¹²In, ⁹⁹mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments". (Chapter 13 in Tumor Imaging: The Radiochemical Detection of Cancer, S.W. Burchiel and B.A. Rhodes, eds., Masson Publishing Inc. (1982)).

For diagnostic purposes, preferably the probes (e.g., antibodies or oligonucleotides) of the invention do not bind to enaptin and do not hybridize with enaptin encoding mRNA, respectively.

The marker Drop1 is also useful for prognosis, for monitoring the progression of an epithelial tumor, for monitoring the therapeutic success during and/or after a tumor therapy, for predictive purposes and the diagnostic evaluation of the degree of malignancy of the tumor (grading and staging), e.g. by using in situ hybridisation, e.g. according to the examples below.

In addition, the inventors have discovered that if the Drop 1 promotor is hypermethylated, Drop 1 will not be expressed or it will be expressed on a lower level (i.e. it is downregulated). Thus, a therapeutic approach is the use of methyltransferase inhibitors. Furthermore, the promotor may be used for epigenomic diagnostic, preferably on an array formate.

The following examples illustrate the invention.

### Example 1

### General methods

### (A) Reaction kits

ABI PRISM-BigDye-Terminator-Cycle-Sequencing-Ready-Reaction-Kit, Version 2 (Applied Biosystems, Weiterstadt, Germany); DIG RNA Labeling Kit (Roche Diagnostics, Mannheim, Germany); DNA Lab Chip Kit (Agilent Technologies, Waldbronn, Germany); PCR Product Purification Kit (Roche Diagnostics); QIAprep Spin Miniprep Kit (Qiagen, Hilden, Germany); QIAquick Gel Extraction Kit (Qiagen); QIA shredder columns (Qiagen); Rneasy Mini Kit (Qiagen); TOPO TA Cloning Kit (Invitrogen, Karlsruhe, Germany); Qiagen PCR Cloning plus kit (Qiagen).

### (B) Enzymes, buffers, reagents and markers

Advantage-cDNA-Polymerase-Mix (Clontech, Heidelberg, Germany); anti-DIG-FAB-Fragments (Boehringer, Mannheim, Germany); BamHI-restriction enzyme and -reactionbuffer (Gibco BRL, Eggenstein, Germany); cDNA PCR-Reaktionsbuffer (Clontech); Desoxyribonuklease I (Gibco BRL); DNTP Mix (Amersham, Cleveland); DTT (Sigma-Aldrich, Deisenhofen, Germany); Dulbeccoís Modified Eagle Medium (DMEM) (Invitrogen, Karlsruhe, Germany); EcoRV-restriction enzyme (Gibco BRL); Fetal calf serum (FCS) (Bio Whittaker, Verviers); First strand buffer (Gibco BRL); DNA size standard: Gene Ruler 100 bp (MBI Fermentas, St. Leon Roth, Germany); Gene Ruler 1 kb (MBI Fermentas); Goldstar DNA-Polymerase (Eurogentec, Karlsruhe, Germany); Goldstar DNA-Polymerase-Buffer (Eurogentec); Phosphate Buffered Saline (PBS) (Invitrogen); Proteinase K (Sigma-Aldrich); RLT Lysis Buffer (Qiagen); RNAse A (Sigma-Aldrich); RNA size standard (Gibco BRL); Rnase inhibitor (Boehringer, Mannheim); RNAlater (ams, Wiesbaden, Germany); SuperSCRIPT II RNase H-Reverse Transcriptase (Gibco BRL); t-RNA (Sigma-Aldrich); Trypan blue (Biochrom, Berlin, Germany); Trypsin (Panbiotech, Aidenbach, Germany).

### (C) Carcinoma cell lines

Cell lines CaOv-3, ES-2, MDAH 2774, NIH:OVCAR-3, SK-OV-3 were obtained from the American Type Culture Collection (Rockville, USA), GG HeST and MT were provided by Dr. M. Lindner, Oncology Laboratory of the Women's University Hospital in Heidelberg and Dr. B. Gückel, Women's University Hospital Tübingen

| | |
|---|---|
| Cell line | Characteristics |
| CaOv-3 | Derived from a human adenocarcinoma of the ovary, grown as monolayer, epithelial -like morphology |
| ES-2 | Derived from a poorly differentiated human ovarian clear cell carcinoma, hyperdiploid karyotype |
| MDAH 2774 | human adenocarcinoma of the ovary, grown as monolayer with epithelial morphology |
| NIH:OVCAR-3 | Derived from the malignant ascites of a patient with progressive adenocarcinoma of the ovary, grown as monolayer with epithelial morphology, exhibits androgen- and estrogen receptors, near triploid karyotype, large number of structurally abnormal and unassignable chromosomes. |
| SK-OV-3 | Derived from from malignant ascites of a patient with adenocarcinoma of the ovary, grown as monolayer; epithelial -like morphology |
| GG | Derived from the malignant ascites of a patient with poorly differentiated serous-papillary cystadenocarcinoma of the ovary, stage FIGO III, GIII, with metastases in the ovary, both fallopian tubes, isthmus of the cervix uteri, perimetrium of the corpus utery, bladder peritoneum and omentum majus. Grown as monolayer. |
| HeSt | Derived from the malignant ascites of a patient with serous-papillary cystadenocarcinoma of the ovary, stage FIGO III, grown as monolayer. |
| MT | Derived from the malignant ascites of a patient with serous-papillary cystadenocarcinoma of the ovary, stage FIGO III, grown as monolayer. |

### (D) Procurement of tissues

Upon agreement of the patient in writing, procurement of tumor and normal tissue was performed intraoperatively under sterile conditions at the Women's University Hospitals in Heidelberg and Tubingen. Epithelia were removed by 10 to 15 smears using a plastic loop. In cases were no healthy ovarian epithelium could be obtained, epithelium from the infundibulum tubae uterinae (the uppermost part of the fallopian tube) was used as a reference tissue. In terms of expression patterns this is the tissue most closely related to the ovarian surface epithelium. Each smear was immediately transferred to a 1.5 ml Eppendorf tube containing 350 µl of RNA-Later. These samples were stored at -80°C.

In addition, tissue blocks of solid carcinoma of tumor free ovarian tissue and of fallopian tube were procured by transferring them into Falcon tubes containing 4% paraformaldehyde buffer and were fixed for in-situ hybridisation. After fixation for 15 h at 4°C, the blocks were washed twice with PBS for 15 min each and stored in 70% ethanol at 4°C. Additional small blocks of tissue were procured for laser captured microdissection in cryotubes containing 350 µl of RNAlater, shock frozen in liquid nitrogen at -196 °C and stored at -80°C. Dehydrated frozen sections of 7 µm were microdissected using an Arcturus PixCell II microdissector.

### (E) RNA isolation, quality control, and reverse transcription

Samples in RNAlater were thawed. Three volumes of RLT lysis buffer containing 10% 2-mercaptoethanol were added, the cells were lysed by incubation for 30 min at 37°C under gentle shaking and finally vortexed. Cell fragments were removed by passing the solution through a QIA shredder column for 2 min at 8,000 rpm in a microcentrifuge. The eluant was mixed with an equal volume of 70% ethanol and centrifuged through RNeasy mini spin columns for 15 sec at 8,000 rpm. The RNA bound to the columns was washed with 700 µl of RW1 buffer and twice with 500 µl RPE buffer containing ethanol. The RNeasy filters were dried by centrifugation for 2 min at maximum speed. RNA was eluted with 2x30 µl RNase free DEPC water at 8,000 rpm. RNA concentration was determined spectrophotometrically and adjusted to equal concentration for each individual pair of tissues. Its quality was assessed by agarose gel electrophoresis or by using an Agilent Bioanalyzer 2100. The ratio of 28S:18S rRNA should be close to 2:1. Residual DNA was digested with 2U of DNAse I for 15 min at 25°C. The reaction was terminated by addition of 1/10 vol. 25 mM EDTA pH 8.0 and denaturation for 10 min at 65°C. In addition, RNA was isolated from frozen tissue sections, microdissected by laser capture microdissection (Arcturus PixCell II). Integrity of the RNA was checked using the Agilent system. From this source, pA-RNA was reversely transcribed and used without amplification for Differential Display and quantitative PCR.

### (F) Differential display

Differential display was performed using a modification of Liang's and Pardee's method with a self-designed set of arbitrary primers. A strong difference in expression was found in patient 31 with the arbitrary decamer primer 5' ACAAGGCACC 3'( designated DOC), and a decamer anchor primer 5' TTTTTTTTVG 3', resulting in a 680 bp PCR fragment in normal epithelium, which was absent from the pattern derived from the tumor of the same patient. This fragment was eluted from the gel, amplified, cloned into TOPORII, and sequenced.

### (G) Validation of Differential Expression by Quantitative and Semi-Quantitative RT-PCR

The differential expression was validated by quantitative PCR and analyzed in the Agilent 2100 Bioanalyzer (Agilent Technologies, Waldbronn, Germany, PC Hewlett packard, Vectra VL, Hewlett packard, Boeblingen; Software Agilent 2100 Bio Sizing 1.3, Agilent Technologies, Waldbronn) or by semiquantitative analysis of the resulting cDNA on a 2% agarose gel.

Conditions:
1 µl cDNA of the 20 µl RT-mixture (corresponding to 0.5 ng of cDNA);
1 µl Drop1 Upper primer (5' CTGGAGAAACGGCTGTCACAAATA 3' corresponding to positions 8255-8278 in exon 53), 20 µM;
1 µl Drop1 Lower primer (Lower primer 5' CCTCTCTACATAGTCATTCCATTGGCTA 3', complementary to positions 8500-8473 in exon 55), 20 µM;
5 µl cDNA-PCR reaction buffer, 10 x;
1 µl dNTP-Mix, 10 mM;
1 µl advantage-cDNA-polymerase mix, 50 x;
40 µl aqua dest.

| advantage-cDNA polymerase mix | | cDNA-PCR reaction buffer | |
|---|---|---|---|
| Glycerol | 1,0 % | Tricine-KOH, pH 9,2, | 40 mM |
| Tris-HCL, pH 7,5, | 0,8 mM | KOAc | 15 mM |
| KCL | 1,0 mM | Mg(OAc)₂ | 3,5 mM |
| (NH₄)₂SO₄ | 0,5 mM | BSA | 3,75 µg/ml |
| EDTA | 2,0 µM | | |
| β-Mercaptoethanol | 0,1 mM | | |
| Thesit | 0,005 % | | |

The PCR reactions were performed in a PTC-200 Peltier Thermal Cycler (Biozym, Hess. Oldendorf, Germany) under the following conditions:

| | | |
|---|---|---|
| 94°C | 4 min | Denaturation of DNA |
| 1. 94°C | 1 min | Denaturation of DNA |
| 2. 58°C | 40sec | Annealing |
| 3. 72°C | 1 min | Elongation |
| 4°C | hold | |

Steps 1 to 3 were repeated 34 to 46 times. After individual cycles in that range, aliquots were removed and run on a gel.

The amount of PCR-product from the first cycle producing a visible band of 246 bp was then quantitated using the Agilent system and an intercalating dye to detect double-stranded DNA. Under these conditions, quantitative PCR detected Drop1 plus enaptin, yet not cpg2, and the various nexins.

### (H) Northern blot analysis and cancer profiling array

Clone 3R9 had been generated in a 3' RACE experiment using an ovarian RACE library. Two human multiple-tissue Northern blots (Clontech) were pre-hybridized in 0,5 M sodium phosphate, pH 7,2, 7% SDS, 1 mM EDTA and 0,1 mg/ml salmon sperm DNA at 65°C for 1 h, and subsequently hybridized for 14 h at 65°C in the same buffer containing the labelled probe at a concentration of 1,5 x 10⁶ cpm/ml. The filters were washed twice at 65°C in a buffer containing 0,2 X SSC and 0,2 % SDS and exposed for 20 h. To confirm an equal loading of mRNA in each lane, the blots were hybridized with labelled human β-actin cDNA.

The cancer profiling array from BD Biosciences Clontech (Palo Alto) catalog # 7841-1 included normalized cDNA from 241 tumor and corresponding normal tissues from individual patients, amplified by SMART technology. Human ubiquitin cDNA control probe had been hybridized to the array to ensure equal loading.

### (I) In situ hybridisation

In situ hybridisation was carried out as described by DeJong et al., 1985; Straus, 1982; Scopsi und Larson, 1986).

### (J) Digoxigenin-labeling of RNA

A digoxigenin-labeled RNA-probe was generated by in vitro-transcription of the 3R9 clone from pCRII-Topo (see above), into which the cDNA fragment had been cloned to produce cDNA for sequencing this product. The vector is suited for in vitro-transcription, as it has promotors for the phage RNA-polymerases SP6 und T7 on either side of the insert. Thus, after linearization at the restriction sites BamHI and EcoRV, two single-stranded RNA-probes of opposite orientation ("sense" and "antisense") could be transcribed.

Linearization was assayed by gel electrophoresis of a small aliquot, and labeling was performed using the DIG-RNA-labeling-kit.

| Digoxigenin-labeling of RNA using SP6-polymerase after EcoRV cleavage | | T7-polymerase after BamHI-cleavage | |
|---|---|---|---|
| linearized vector-DNA 7,4 µl | (1 µg) | linearized vector-DNA 5µl | (1 µg) |
| transcription buffer , 10x, | 2 µl | transcription buffer, 10x | 2 µl |
| Labeling-Mix, 10x | 2 µl | Labeling-Mix, 10x | 2 µl |
| RNase-Inhibitor, 20 U | 1 µl | RNase-Inhibitor, 20 U | 1 µl |
| SP6-polymerase, 40 U | 2 µl | T7-polymerase, 40 U | 2 µl |
| DEPC-H₂O | 5,6 µl | DEPC-H₂O | 8 µl |

The components were mixed on ice and incubated for 2 h at 37°C. To assess the quality and the amount of the resulting single-stranded RNA, 1 µl of the product was run on a 2% TAE-agarose gel. Then, 2 µl (= 2U) RNase-free DNase I was added and incubated at 37°C for 15 min, to destroy the vector and avoid disturbance of the hybridisation by unlabeled DNA. The reaction was stopped by adding 2 µl of 0,5 mM EDTA pH 8, and the probes were stored at -20°C.

### (K) Preparation of tissue sections

Procured tissues that had been stored in 70% ethanol/PBS were washed in 10% Ethanol/PBS at 25°C and dehydrated by subsequent washes in 85%, 95% ethanol/PBS, 3x 100% ethanol at 25°C, 2 x in xylol, and incubated in xylol for 15 h at 25°C. Subsequently the tissues were incubated for 60 min at 56°C in a paraffin/xylol-mixture, and covered with pure paraffin for 3x for 60min in a Tissue Tek TEC (Vogel, Gießen). Storage was at 4°C.

Sections of 7 µm were taken on a microtome (Leica, Nussloch, Germany), placed on silanized glass slides and dried for 15 h at 40°C. To remove the paraffin, the tissues were transferred into xylol and rehydrated in seven successive steps for 2 min each in 100%, 95%, 90%, 80%, 70%, 50% and 30% ethanol, 5 min in 0,9% NaCl, 5 min in PBS-buffer. After fixation for 20 min in 4% paraformaldehyde/ PBS the sections were washed 2 x 5min in PBS, digested with with RNase-free proteinase K (20 µg/ml) at 37°C for 20 min, washed again in PBS for 5 min. Excess paraformaldehyde was blocked by acetylation with 0,625 ml acetic anhydride in 3,125 ml triethanolamine and 250 ml H₂O for 10 min, and the slides were finally washed for 5 min in PBS. To reduce background, the sections were prehybridized for 1-2h in hybridisation solution in the absence of the probe.

### (L) Hybridisation solution

10x stock solution of salts: 0,2% polyvinylpolypyrrolidone; 0,2% Ficoll 400; 1,38% NaH₂PO₄, pH 6,8; 50 mM EDTA; 3 M NaCl; 0,1 M Tris, pH 8,0.

100 µl of this stock solution were mixed with 500 µl of formamide, 2 µl 10 mg/ml t-RNA, 200 µl of 50% dextran sulfate, 100 µl 1 M 1,4-dithiothreitol, and 100 µl H₂O.

### (M) Preparation of probes

To remove contaminating free labeled nucleotides from the digitonin-labeled RNA probes, 20 µl aliquots of probe were precipitated by addition of 2,4 µl 4M LiCl and 75 µl 100% ethanol and incubation at -70°C for 15 h. The precipitate was spun down in a centrifuge, the supernatant removed, and the probe washed in 50 µl of 70% ethanol. The sediment was centrifuged, air-dried and taken up in the hybridisation solution to a final concentration of 5 µg/ ml. The probe was denatured for 7 min at 70°C and applied to the tissue sections. In parallel, a known labeled probe was applied to control sections.

### (N) Hybridisation

Prehybridisation buffer was cautiously removed from the slides. 100 µl of hybridisation solution containing the digitonin-labeled RNA probe was applied to each section. Slides were incubated in a moist chamber for 15 h at 45°C.

### (O) Posthybridisation

Solutions:
SSC 88,2 g Na-citrate x 2H₂O 175,325 g NaCl in 1000 ml H₂O, adjusted to pH 7.0 with HCl;
2 x SSC; 2 x SSC/50% formaledehyde; 5 x SSC and 5x SSC/ 50% formaldeyhde were prepared accordingly:
NTE-buffer 100 ml 5 M NaCl, 10 ml 1 M Tris pH 8,0, 100 ml 0,5 M EDTA, 790 ml H₂O;
B1 DIG 100 ml 1 M Tris pH 7,5, 30 ml 5 M NaCl, 870 ml H₂O;
Blocking solution 50 ml B1 DIG, 0,5 g blocking reagent, 2 mM Levamisol;
Anti-DIG-FAB-fragment 1: 500 dilution in 4 ml B1 DIG;
NTMT 10ml 5 M NaCl, 50 ml 1 M Tris-HCl pH 9,5, 25 ml 1 M MgCl₂, 415 ml H₂O, 500 µl Tween 20;
Staining solution amount corresponding to anti-DIG-FAB-fragment: NTMT, 2 mM Levamisol, BCIP (5-bromo-4-chloro-3-indolylphosphate), NBT (Nitroblue Tetrazolium)

The hybridisation solution was removed from the slides, the sections were washed 2 x 5 min in 5xSSC at 25°C, 30 min with 5x SSC/50% formamide while the temperature increased from 37°C to 60°C, and 60 min in 2x SSC/50% formamide. To remove unbound single-stranded RNA-probe, the sections were then incubated for 15 min at 37°C in NTE-buffer, unbound probe was digested for 30 min at 37°C with RNAse A (20 µg/ml) in NTE-buffer, followed by a wash for 15 min at 25°C in NTE-buffer, and 2xSSC for 30 min at 25°C.

The tissue sections were now treated for 15 min with B1 DIG at 25°C. The reaction was terminated by blocking solution for 60 min at 25°C. The blocking solution was removed, anti-DIG-FAB-fragments in blocking solution were added and incubated for 15 h at 4°C.

The sections were washed for 10 min with B1 DIG and NTMT with 2 mM Levamisol at 25°C. Staining solution was added and the slides were incubated in the dark. A red-brown staining results. When the desired intensity is reached, the reaction is stopped by washing with PBS. For conservation, the slides were treated 2 x 5min with 70% and 90% ethanol, followed by 2 x 10min 100% ethanol and xylol. Finally the stained sections were covered with Entellan. Micrographs were viewed and documented using the foto microscope of the Arcturus microdissector.

### (P) Treatment of cells with methyltransferase inhibitors

Treatment of cells with methyltransferase inhibitors was carried out as described by Widschwendter et al., J.Nat.Cancer Inst. 92 (2000), 826-832). All-*trans*-retinoic acid (ATRA) and 5-aza-deoxycytidine (Aza-CdR) were from Sigma Chemical Co. (St. Louis, MO). 1 mM solutions were prepared in dimethyl sulfoxide and diluted in culture medium. Cells were incubated either for 5 days, with 2.5 mM Aza-CdR, for 24 h with 1 mM ATRA, or for 5 days with 2.5 mM Aza-CdR followed by 24 h with 1 mM ATRA, or left untreated. They were detached with trypsin (0.05%)-EDTA (0.02%) in Dulbecco's phosphate-buffered saline (PBS), washed, and pelleted.

### (Q) Cloning of the GFP- Drop1 aminoterminal domain fusion construct for expression in mammalian cells

The 5' region of the ORF, encoding aar 1 - 396 (Drop1) the two calponin homology domains, was amplified using the primers 5'-GTCGACGATGGCAACCTCCAGAGGGGCCTCCCG-3' with a Sal I restriction site as upper primer (U278) and 5'-GGGCCCTGGTGCCCAGAGGTGCAGGAAGAGA-3' with an Apa I restriction site as lower primer (L1492) and a previously sequenced overlap clone (H) on the basis of cDNA from the SKOV-3 cell line as template (annealing at 68 øC, 25 cycles). The PCR product was cloned into pDrive for amplification and sequencing, cut out with Sal I and Apa I, and cloned into pEGFP-C3 (Clontech, GenBank Accession # U57607), behind a GFP-domain. Correct insertion was confirmed by sequencing. 5-10 x 104COS-7 cells/well were grown in DMEM, containing 10% fetal calf serum, 1% L- glutamine and 1% nonesssential amino acids, for 24 h in Lab-Tek chambered cover glasses, transfected with 0,75 - 1.5 _g of DNA/ well mixed with 3 to 6 _1 FuGENE 6 (Roche). After 24 h cells were observed under a Zeiss Axiovert S100 TV microscope (excitation at 485 nm, filter 485/20, - emission at 515 nm, filter 530/30, dichroic mirror 505) using a 20x objective and a Plan-Neofluar oil objective with 63x magnification. Fluorescence images were recorded for 100 to 250 msec with 30% gain.

### (R) Cloning of the carboxyterminal spectrin-like domain

The carboxyterminal spectrin-like domain of Drop1 was selected as an antigen. cDNA encoding that region was obtained using cDNA from CAOV-3 tumor cells as template, 5'-GATATCATGTATTTAGATGCGGTCCACGAGTTC-3', containing an EcoR V restriction site as an upper primer, and 5'-GTCGACTGGGGACCCTTGATTTAGAGGTTAACTT-3', containing a Sal I restriction site as lower primer. This primer binds to a sequence in the 3' UTR of Drop1, which in the enaptin gene is located in a putative intron. The PCR product (annealing at 64 øC, 45 cycles) was cloned into TopoTA for amplification and sequencing, cut out with EcoR V and Sal I, and cloned into pET32a for expression in E. coli BL21(DE3).

### (S) Expression of the recombinant protein in E. coli and its purification from cell lysates

Bacteria were induced at 25° C for 3 h with 1 mM IPTG, harvested by low-speed centrifugation, washed in PBS containig protease inhibitor cocktail (Roche), swollen on ice in hypotonic 50 mM sodium phosphate pH 8.0 containing 1 mg/ ml lysozyme, supplemented by EDTA-free protease inhibitor cocktail, and lysed by ultrasonication. DNA was digested with 1 U/ml of benzonase (Merck) for 30 min at 25 øC. Upon addition of 300 mM NaCl the lysate was centrifuged in an ultracentrifuge at 226,000 x g for 60 min. The His-tag-containing proteins in the supernatant were bound to Ni-NTA Superflow in a column of 1,6 cm _ x 10 cm (Qiagen). Unbound material was removed by 50 mM Na phosphate, 300 mM NaCl, 20 mM imidazole, and the bound protein was eluted with the same buffer containing 150 mM imidazole. Fractions of 1 ml were taken and aliquots were checked for protein bands by gel electrophoresis.

Fractions of interest were pooled, diluted ten-fold with 25 mM Tris.HCl pH7.5, loaded onto a MonoQ HR 5/5 column 0.5 cm _ x 5 cm (Amersham Bioscience) and a gradient was run from the loading buffer with 30 mM NaCl to the same buffer containing 1M NaCl. The relevant fractions were pooled, concentrated, and the buffer was changed to 50 mM Tris pH 8.0, as required for cleavage with enterokinase (Invitrogen). With10 U of this protease/ mg of protein the thioredoxin fused to the C-terminal Drop1-fragment was cleaved for 16 h at 8 φC. The enterokinase was removed by EK-Away resin (Invitrogen). The solution was diluted with 50 mM sodium phosphate containing 500 mM NaCl pH 8.0 as the starting buffer for a Ni-NTA column (1 cm _ x 5.5 cm), to which the thioredoxin containing the His-tag was bound, while the C-terminal Drop1 domain remained in the flowthrough. It was concentrated in a Savant Speedvac Plus vacuum centrifuge and for the immunization of mice and rabbits the buffer was changed by passage over a NAP25 column (Pharmacia). Purity and quantity of the protein (0.6 mg/ml) were checked by gel electrophoresis on a 15% Kornberg gel.

### (T) Generation of monoclonal antibody DROP1-2.9

Female BALB/c mice were immunized with the purified C-terminal spectrin-like domain.

For the first immunization the protein emulsified in complete Freund_s adjuvant was injected subcutaneously at 4 sites at the flank. Additional five immunizations were given intraperitoneally in intervals of two weeks consisting of the purified protein diluted in sterile PBS. Three days after the last challenge, immune spleen cells were fused to Ag8.653 mouse myeloma cells according to standard procedures.

Supernatants of growing hybridomas were screened for binding to the protein by enzyme-linked immunosorbent assay (ELISA). Positive samples were further analyzed by immunocytology using cytocentrifuge preparations of SK-OV-3 ovarian carcinoma cells. Thus, hybridoma DROP1-2 was identified and subsequently cloned by limiting dilution. Clone DROP1-2.9 was selected according to the same assays mentioned above. It secretes an IgG1 kappa antibody as demonstrated by a mouse isotype-specific ELISA.

### (U) Staining of cytospins of SKOV-3 cells

SKOV-3 cells were spun down on a coverslip and . permeabilized and fixed for 10 min at -20 øC in acetone. After drying they were incubated with peroxidase-coupled anti-mouse IgG-Fc for 30 min at 25 øC: A stock solution of 8 mg/ ml 3-amino-9-ethylcarbazole (Sigma) in dimethyl formamide was prepared.

For use it was diluted 20-fold with 0.1 m Na acetate pH 5.2 and 2 _1/ml of H2O2 were added This solution was used for generating a red stain with the peroxidase within 5 - 10 min. The reaction was stopped by two washes with PBS.

### Example 2

### Differential display PCR

In an analysis of differential gene expression of ovarian carcinomas a 691 bp cDNA fragment was found which was downregulated in an ovarian carcinoma (Figure 1). The band labeled with an arrow shows a PCR product in normal tissue absent from the tumor. A specifically designed arbitrary primer and a G-anchor primer had been used for this PCR. The product was eluted from the gel, re-amplified, cloned into the TOPO TA vector and sequenced.

### Example 3

### Validation of differential display data by quantitative PCR using cDNA from tissues of patients

Using gene specific primers, normal and ovarian carcinoma tissue of eight individual patients was analyzed by quantitative PCR for differences in expression (Table 1). In all patients Drop1 was expressed at a higher level in the healthy tissue, the ratio of expression between normal tissue and carcinoma varying from 14 : 1 to 210 : 1. In addition, it was shown that healthy fallopian tube epithelium is, in this case, a suitable reference tissue for analyzing expression differences in ovarian carcinomas, since microdissected epithelium cells from healthy fallopian tube and ovarian surface epithelium express Drop1 at a comparable level. The ratio of fallopian tube to ovarian surface epithelium was 0.95.

**Table 1**

| **Drop1 expression level in tissue pairs from individual** **patients and in ovarian carcinoma cell lines** | | | | | |
|---|---|---|---|---|---|
| patient | normal | | Ratio N:C | OVCA cell lines | |
| | microdissected | | | | |
| 82-84 | tube epithelium 20 nM | ovarian surface epithelium 21 nM | 0.95 | NIH:OVCA R-3 | 43 pM |
| | cDNA from swabs normal | carcinoma | | MDAH 2774 | 34 pM |
| 30 | 336 nM | 5.8 nM | 58 | CAOV-3 | 5.0 nM |
| 31 | 1020 nM | 6,9 nM | 148 | SKOV-3 | 11 nM |
| 44 | 94 nM | 0.65 nM | 140 | HEST | 0.26 nM |
| 48 | 320 nM | 23 nM | 14 | ES-2 | 51 pM |
| 50 | 300 nM | 2.3 nM | 130 | GG | 19 pM |
| 53 | 928 nM | 4.4 nM | 210 | MT | 66 pM |
| 93 | 3.4 nM | Neg | 130 | | |
| 100 | 19 nM | Neg | 48 | | |
| | microdissected | | | | |
| BL | ductal epithelium 8.5 nM | ductal invasive carcinoma 8.5 nM | 1.0 | MCF-7 | 20 pM |
| | | | | HeLa | 5,3 nM |
| Calculated to the level after 38 PCR cycles; concentration expressed as nM. | | | | | |

### Example 4

### Expression of Drop1 in tumor cell lines and in frozen sections from tumors

Ovarian carcinoma cell lines were also analyzed for Drop1 expression (Table 1). Quantitative PCR with pairs of tissues from individual patients was performed with normalized cDNA derived without amplification from healthy tube epithelium and ovarian carcinoma. As expected, expression was low in most cell lines. However, two cell lines, SKOV-3 and CAOV-3 had a normal expression level of approximately 5 nM after 38 PCR cycles. In addition, cDNA was prepared from frozen sections of ovarian and mammary carcinomas from individual patients. In these specimens, Drop1 was downregulated in 6 out of 12 ovarian carcinomas and in eight out of 16 mammary carcinomas (Table 2).

**Table 2**

| **Drop1 expression levels in frozen sections of tumors from** **individual patients** | | | |
|---|---|---|---|
| Patient | Drop level | patient | Drop1 level |
| | | | |
| Ovarian CA | | Mammary CA | |
| 829 | 2.7 nM | 1170 | 0.020 nM |
| 895 | 3.1 nM | 1177 | neg. |
| 897 | 0.011 nM. | 1179 | neg. |
| 1111 | 3,0 nM | 1181 | 150 nM |
| 1117 | neg | 1182 | neg |
| 1130 | 7.2 nM | 1184 | neg |
| 1131 | 0.005 nM | 1188 | 31 nM |
| 1160 | 3.8 nM | 1189 | 24 nM |
| 1161 | 0.012 nM | 1191 | 11 nM |
| 1172 | 0.019 nM | 1193 | neg. |
| 1174 | 0.014 nM. | 1194 | neg. |
| 1178 | 3.8 nM | 1195 | neg. |
| | | 1198 | 3.0 nM |
| | | 1202 | 5.3 nM |
| | | 1203 | 4.0 nM |
| | | 1205 | 4.1 nM |
| | | | |
| Results of quantitative PCR using normalized cDNA without amplification from ovarian carcinoma cell lines, from frozen ovarian carcinomas and from mammary carcinomas; drop level calculated for 38 cycles. | | | |

### Example 5

### Cancer profiling array

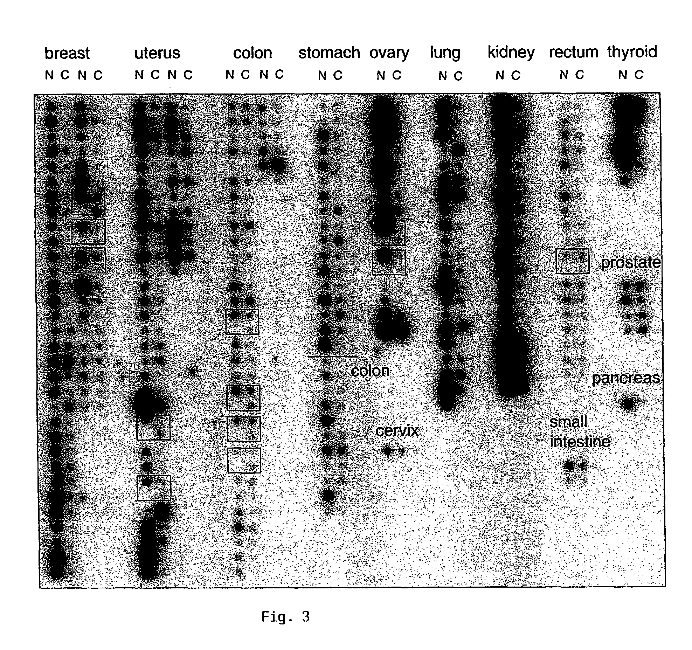

Using a cancer profiling array, the differential expression of Drop1 was analysed in pairs of tissues in an additional 14 patients (Figure 3). On this array, cDNA from normal ovarian tissue (N) and carcinoma tissue (C) has been spotted in an amount normalized to equal ubiquitin cDNA concentration. The tumors were 12 adenocarcinomas, one mucinous borderline tumor and one clear cell carcinoma. Upon hybridisation with the radioactive Northern probe, in 12 patients the expression of Drop1 was distinctly lower in the tumor. For two patients, cDNA prepared from metastatic tissue was also spotted and featured a similar level of expression as the corresponding primary tumor.

Tissue pairs from patients with other types of carcinomas were likewise analyzed for Drop1 expression. It was significantly lower than in the corresponding normal tissue in 85% of the mammary carcinomas, stomach, lung, kidney and thyroidea carcinomas. Colon and rectum carcinomas have a low expression level with a less distinct pattern, and for cervix, pancreas, and small intestine the number of cases is not representative.

### Example 6

### Regulation of Drop1 by methylation of the promoter

The possibility that promoter methylation might downregulate Drop1 was investigated. Treatment of cell lines exhibiting a low level of Drop1 with the demethylating agents AZA (5-aza-cytidine ) and ATRA (all-trans-retinoic acid) resulted in an increase of Drop1 mRNA by an order of magnitude (Table 3). No increase of expression was observed in SKOV-3, a cell line with a physiological level of Drop1. This indicates that the lower level of Drop1 expression in cell lines GG and M130 was caused by methylation of the Drop1 promoter.

**Table 3**

| **Expression of downregulated Drop1 in cells treated with** **inhibitors of gene methylation** | | | | |
|---|---|---|---|---|
| Cells | in medium | in Aza | in ATRA | in Aza+ATRA |
| GG | 5,2 x 10⁻⁵ | 4,7 x 10⁻⁵ | 1,3 x 10⁻⁴ 2.5fold | 7,7 x 10⁻⁴ 15fold |
| M130 | 1,0 x 10⁻³ | 1,0 x 10⁻³ | 1,8 x 10⁻² 18fold | 5,6 x 10⁻⁴ |
| SKOV-3 | 2,2 x 10⁻⁴ | 2,4 x 10⁻⁴ | 2,2 x 10⁻⁴ | 1,9 x 10⁻⁴ |
| Aza: 5'-aza-2'-deoxycytidine, ATRA: all-trans-retinoic acid Ratios are [31N/GDOC/1], measured in the LightCycler [G3PDH] | | | | |

### Example 7

### In situ hybridisation

Localization of the mRNA of Drop1 in healthy ovarian tissue, fallopian tube tissue and tumor tissue was performed by in-situ hybridisation (Figure 4). Drop1 was specifically expressed in epithelium of the healthy ovary and at a comparable level in the epithelium of a healthy fallopian tube. Other cell types of these organs do not express Drop1. This figure again shows that tube epithelium in this case is a suitable reference tissue and that the difference in expression of Drop1 is not based on the characteristics of the tube epithelium.

### Example 8

### Multi-tissue-Northern blot

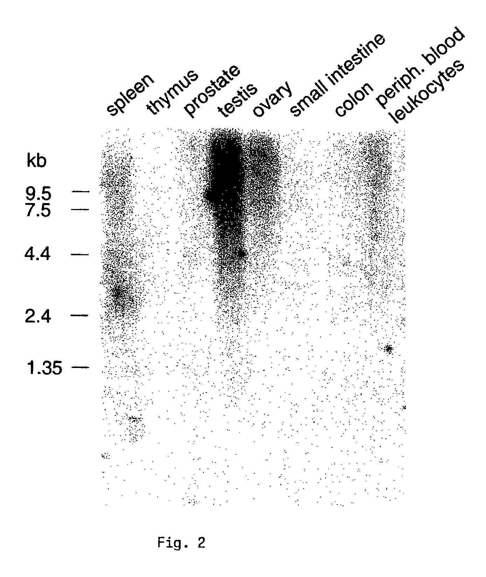

It could be demonstrated (Figure 2), that Drop1 mRNA is present in testes (Figure 2; slot 4) and in ovary (Figure 2; slot 5) in a multi tissue Northern Blot. The remaining tissues yielded only a weak signal or nor signal at all. The smear may be caused by differentially used exons resulting in mRNAs of different lengths.

### Example 9

### Completion and characteristics of the Drop1 sequence

The sequence of the differential display fragment had featured an open reading frame. By "RACE-walking" using a commercial ovarian RACE library and by validating the resulting RACE clones using overlap PCR on the basis of one ovarian carcinoma cell line that did express the Drop1 fragment, an open reading frame of 9,099 bp (Fig. 6B; sequence details) could be completed. The encoding gene comprises 57 exons in chromosome region 6q25. Upon completion of this sequence a cDNA with a much larger ORF for enaptin was submitted to the data bases (Accession no. AF535142). Exon usage for Drop1, however, was different from that of enaptin in that (a) exon 5 is 36 bp longer than predicted, (b) exon 7 (21 bp) is not used and (c) exon 12 is used in a commercial ovarian cDNA library, yet not in the ovarian carcinoma cell line CAOV-3. It contains the start codon for a putative human homologue of rat cpg2. Exon 58 is longer in Drop1 than predicted, In all clones derived from OVCA-R3 it contains a stop codon immediately following the predicted transition to exon 59. 2,5 kb of the genomic sequence following this stop codon are found in the Drop1 mRNA as 3'UTR. In contrast, an ovarian RACE library yielded clones for both variants: an extended exon 58 with the stop codon for Drop1 as well as the transition to exon 59.

Drop1 encodes a protein of 349 kDa (Figure 10) that is structurally similar to dystrophin. At the N-terminus it features 2 calponin homology domains followed by an ATPase-domain and 12 spectrin-like domains (Figure 7). The homologues of these structures are known to bind other intracellular or transmembrane proteins.

### Example 10

### Cloning and expression of a Drop1-GFP fusion construct

The 5' fragment, Drop1α, containing the two calponin homology domains of the open reading frame, was cloned into the eukaryotic expression vector pEGFP-C3. Transfected cells show a distinct fluorescence of nuclei (Figure 8), suggesting a nuclear association of the N-terminal region.

### Example 11

### Raising of a monoclonal antibody and detection of the antigen in cells and on immunoblots

A C-terminal recombinant protein fragment, amino acid residues 2827 - 3032, was produced in bacteria, isolated and purified to serve as an antigen for generation of a monoclonal antibody. A clone that recognized the recombinant protein on an immunoblot also identified a high molecular weight band in cell lysates of SKOV-3 and stained nuclei of the same cells (Figure 9). Additional smaller protein bands in the pellet of the cells presumably are Drop1 degradation products.

## Claims

1. A nucleic acid molecule encoding the human dystrophin-related polypeptide Drop1 or a polypeptide exhibiting a biological property of Drop1, which is:
(a) a nucleic acid molecule encoding a Drop1 polypeptide that consists of the amino acid sequence as depicted in Figure 10;
(b) a nucleic acid molecule consisting of the nucleotide sequence as depicted in Figure 6B;
(c) a nucleic acid molecule the sequence of which differs from the sequence of a nucleic acid molecule of (a) or (b) due to the degeneracy of the genetic code;
(d) a nucleic acid molecule complementary to the nucleic acid molecule specified in (a) to (c).

2. A recombinant vector containing the nucleic acid molecule of claim 1.

3. The recombinant vector of claim 2 wherein the nucleic acid molecule is operatively linked to regulatory elements allowing transcription and synthesis of a translatable RNA in prokaryotic and/or eukaryotic host cells.

4. A recombinant host cell which contains the recombinant vector of claim 2 or 3.

5. The recombinant host cell of claim 4, which is a mammalian cell, a bacterial cell, an insect cell or a yeast cell.

6. A non-human transgenic animal **characterized by** loss of Drop-1 function.

7. The transgenic non-human animal of claim 6 further comprising at least one inactivated wild type allele of the corresponding Drop1 encoding gene.

8. A polypeptide exhibiting a biological property of the human dystrophin-related polypeptide Drop1 or the related polypeptide which is encoded by a nucleic acid molecule of
claim 1.

9. A method of making a polypeptide exhibiting a biological property of the human dystrophin-related polypeptide Drop1 comprising:
(a) culturing the recombinant host cell of claim 4 or 5 under conditions such that said polypeptide is expressed; and
(b) recovering said polypeptide.

10. A polypeptide produced by the method of claim 9.

11. An antibody that binds specifically to the polypeptide of claim 8 or 10.

12. The nucleic acid molecule of claim 1, the polypeptide of claim 8 or 10, or the antibody of claim 11 which is detectably labeled.

13. The nucleic acid molecule, the polypeptide or the antibody of claim 12, wherein the label is a radioisotope, a bioluminescent compound, a chemiluminescent compound, a fluorescent compound, a metal chelate, or an enzyme.

14. A method for identifying activators/agonists of the human dystrophin-related polypeptide Drop1 comprising the steps of:
(a) incubating a candidate compound with a polypeptide of claim 8 or 10;
(b) assaying a biological activity; and
(c) determining if a biological activity of said polypeptide has been altered.

15. A pharmaceutical composition comprising:
(A) a nucleic acid molecule comprising
(a) a nucleic acid molecule encoding a Drop1 polypeptide that comprises the amino acid sequence as depicted in Figure 10;
(b) a nucleic acid molecule comprising the nucleotide sequence as depicted in Figure 6B;
(c) a nucleic acid molecule encoding a polypeptide the amino acid sequence of which shows at least 65% idendity to the amino acid sequence of the polypeptide encoded by a nucleic acid molecule specified in (a) or (b);
(d) a nucleic acid molecule the sequence of which differs from the sequence of a nucleic acid molecule of (a) to (c) due to the degeneracy of the genetic code;
(e) a nucleic acid molecule, which represents a fragment of a nucleic acid molecule specified in (a) to (d); or
(f) a nucleic acid molecule complementary to the nucleic acid molecule specified in (a) to (e),
(B) a polypeptide exhibiting a biological property of the human dystrophin-related polypeptide Drop1 or the related polypeptide which is encoded by a nucleic acid molecule of (A) above;
(C) a recombinant vector containing the nucleic acid molecule of (A) above; or
(D) an antibody that binds specifically to the polypeptide as defined under (B) above,
and
optionally a pharmaceutically acceptable excipient, diluent or carrier.

16. Use of a nucleic acid molecule as defined in claim 15, polypeptide as defined in claim 15, a recombinant vector as defined in claim 15, an antibody as defined in claim 15 or an activator/agonist obtainable by the method of claim 14 for the preparation of a medicament for treatment of a tumor.

17. A diagnostic kit or array useful for the detection and/or characterization of a tumor or a predisposition to such a tumor, containing a nucleic acid molecule as defined in claim 15, a polypeptide as defined in claim 15 or an antibody as defined in claim 15.

18. Use of a nucleic acid molecule as defined in claim 15, a polypeptide as defined in claim 15 or an antibody as defined in claim 15 for the preparation of a diagnostic composition for diagnosing and/or characterizing a tumor or a predisposition to such a tumor.

19. Use according to claim 16 or 18, wherein the tumor is an ovarian, mammary, stomach, kidney, thyroid, cervical, pancreas, testis or lung carcinoma.

20. Use of an vector as defined in claim 15 for gene therapy.
